(19) Europäisches Patentamt
European Patent Office
Office européen des brevets

(11) Veröffentlichungsnummer: **0 511 569 A1**

# (12) EUROPÄISCHE PATENTANMELDUNG

(21) Anmeldenummer: **92106779.9**

(51) Int. Cl.[5]: **C07D 249/12**, A01N 43/653

(22) Anmeldetag: **21.04.92**

(30) Priorität: **30.04.91 DE 4114074**

(43) Veröffentlichungstag der Anmeldung:
**04.11.92 Patentblatt 92/45**

(84) Benannte Vertragsstaaten:
**BE CH DE DK ES FR GB IT LI NL**

(71) Anmelder: **BAYER AG**

**W-5090 Leverkusen 1 Bayerwerk(DE)**

(72) Erfinder: **Kuhnt, Dietmar, Dr.**
**Körnerstrasse 5**
**W-5090 Leverkusen 1(DE)**
Erfinder: **Müller, Klaus-Helmut, Dr.**
**Bockhackstrasse 55**
**W-4000 Düsseldorf 13(DE)**
Erfinder: **Findeisen, Kurt, Prof. Dr.**
**Dünfelder Strasse 28**
**W-5090 Leverkusen 1(DE)**
Erfinder: **König, Klaus, Dr.**
**Zum Hahnenberg 40**
**W-5068 Odenthal(DE)**
Erfinder: **Lürssen, Klaus, Dr.**
**August-Kierspel-Strasse 145**
**W-5060 Bergisch Gladbach 2(DE)**
Erfinder: **Santel, Hans-Joachim, Dr.**
**Grünstrasse 9a**
**W-5090 Leverkusen 1(DE)**
Erfinder: **Schmidt, Robert R., Dr.**
**Im Waldwinkel 110**
**W-5060 Bergisch Gladbach 2(DE)**

(54) **Substituierte Triazolinone.**

(57) Die Erfindung betrifft neue, substituierte Triazolinone der allgemeinen Formel (I)

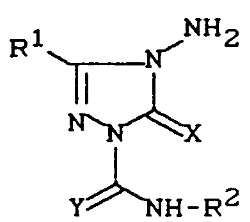

(I)

in welcher

$R^1$ für Alkyl oder Cycloalkyl steht,

$R^2$ für gegebenenfalls durch Aryl, Arylalkyl, Arylalkenyl oder Arylalkinyl substituiertes Cycloalkyl steht, oder für einen Rest der Formel

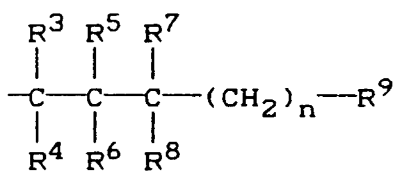

EP 0 511 569 A1

steht,

X für Sauerstoff oder Schwefel steht und

Y für Sauerstoff oder Schwefel steht,
wobei

$R^3$, $R^4$, $R^5$, $R^6$, $R^7$ und $R^8$ für Wasserstoff oder Alkyl stehen,

$R^9$ für jeweils gegebenenfalls substituiertes Cycloalkyl, Aryl oder Heteroaryl steht und

n für eine Zahl 0, 1, 2 oder 3 steht,

wobei jedoch Verbindungen, bei denen gleichzeitig $R^1$ für einen Methylrest, X für Sauerstoff, Y für Sauerstoff und $R^2$ für einen Rest der Formel

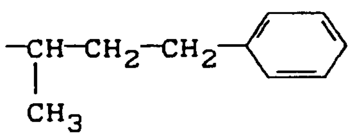 ; 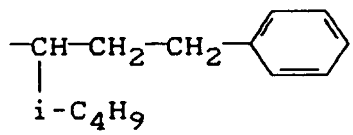 ;

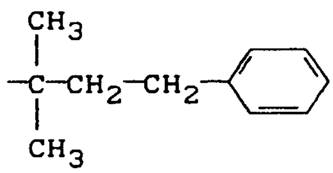 oder 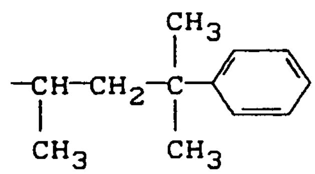

steht, ausgenommen sind, mehrere Verfahren und neue Zwischenprodukte zu ihrer Herstellung und ihre Verwendung als Herbizide.

Die Erfindung betrifft neue substituierte Triazolinone, mehrere Verfahren und neue Zwischenprodukte zu ihrer Herstellung und ihre Verwendung als Herbizide.

Es ist bekannt, daß bestimmte substituierte Triazolinone wie beispielsweise die Verbindung 1-(4-Phenyl-2-butyl-aminocarbonyl)-3-methyl-4-amino-1,2,4-triazolin-5-on herbizide Eigenschaften besitzen (vergleiche z. B. DE-OS 37 19 575).

Die herbizide Wirksamkeit dieser vorbekannten Verbindungen gegenüber Problemunkräutern ist jedoch ebenso wie ihre Verträglichkeit gegenüber wichtigen Kulturpflanzen nicht in allen Anwendungsgebieten völlig zufriedenstellend.

Es wurden nun neue substituierte Triazolinone der allgemeinen Formel (I) gefunden,

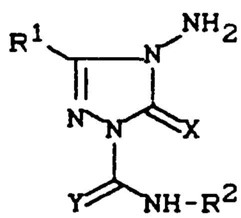

(I)

in welcher

$R^1$ für Alkyl oder Cycloalkyl steht,

$R^2$ für gegebenenfalls durch Aryl, Arylalkyl, Arylalkenyl oder Arylalkinyl substituiertes Cycloalkyl steht, oder für einen Rest der Formel

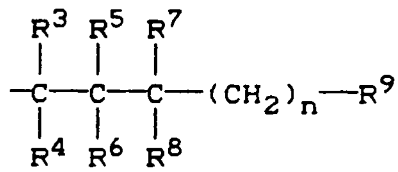

steht,

X für Sauerstoff oder Schwefel steht und

Y für Sauerstoff oder Schwefel steht,

wobei

$R^3$, $R^4$, $R^5$, $R^6$, $R^7$ und $R^8$ für Wasserstoff oder Alkyl stehen,

$R^9$ für jeweils gegebenenfalls substituiertes Cycloalkyl, Aryl oder Heteroaryl steht und

n für eine Zahl 0, 1, 2 oder 3 steht,

wobei jedoch Verbindungen, bei denen gleichzeitig $R^1$ für einen Methylrest, X für Sauerstoff, Y für Sauerstoff und $R^2$ für einen Rest der Formel

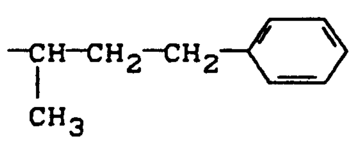 ; 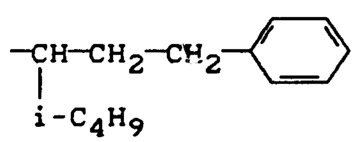 ;

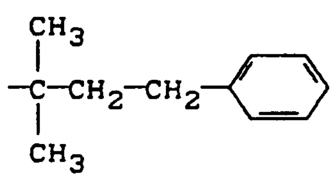 oder 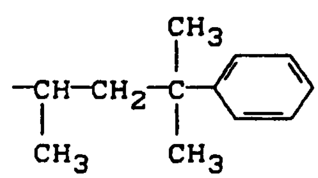

steht, ausgenommen sind.

Die Verbindungen der Formel (I) können gegebenenfalls in Abhängigkeit von der Art der Substituenten

$R^1$ und $R^2$ als geometrische und/oder optische Isomere oder Isomerengemische unterschiedlicher Zusammensetzung vorliegen. Sowohl die reinen Isomeren als auch die Isomerengemische werden erfindungsgemäß beansprucht.

Weiterhin wurde gefunden, daß man die neuen substituierten Triazolinone der allgemeinen Formel (I),

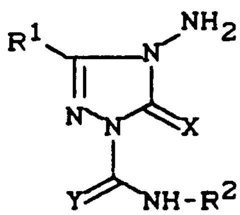

(I)

in welcher

| | |
|---|---|
| $R^1$ | für Alkyl oder Cycloalkyl steht, |
| $R^2$ | für gegebenenfalls durch Aryl, Arylalkyl, Arylalkenyl oder Arylalkinyl substituiertes Cycloalkyl steht, oder für einen Rest der Formel |

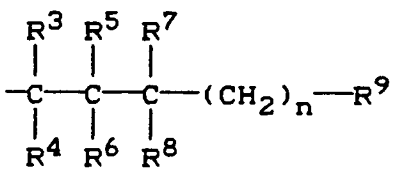

steht,

| | |
|---|---|
| X | für Sauerstoff oder Schwefel steht und |
| Y | für Sauerstoff oder Schwefel steht, |
| | wobei |
| $R^3$, $R^4$, $R^5$, $R^6$, $R^7$ und $R^8$ | für Wasserstoff oder Alkyl stehen, |
| $R^9$ | für jeweils gegebenenfalls substituiertes Cycloalkyl, Aryl oder Heteroaryl steht und |
| n | für eine Zahl 0, 1, 2 oder 3 steht, |

wobei jedoch Verbindungen, bei denen gleichzeitig $R^1$ für einen Methylrest, X für Sauerstoff, Y für Sauerstoff und $R^2$ für einen Rest der Formel

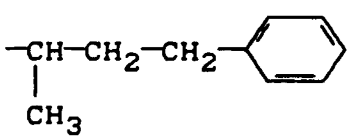

;

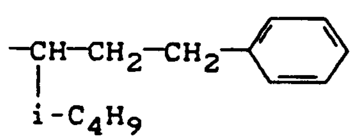

;

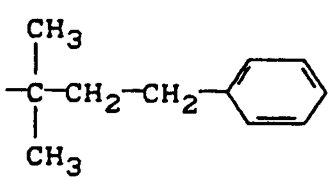

oder

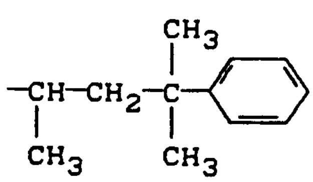

steht, ausgenommen sind,
erhält, wenn man

a) Hydrazone der Formel (II),

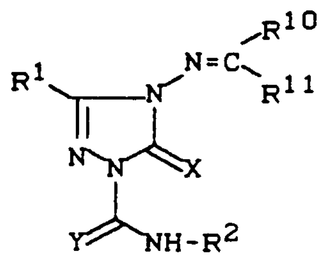

(II)

in welcher

$R^1$, $R^2$, X und Y die oben angegebene Bedeutung haben und

$R^{10}$ und $R^{11}$ unabhängig voneinander jeweils für Wasserstoff, Alkyl, Aralkyl oder Aryl stehen,

mit einer Säure gegebenenfalls in Gegenwart eines Verdünnungsmittels umsetzt,

oder wenn man

b) 1H-Triazolinone der Formel (III),

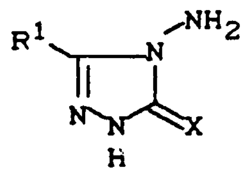

(III)

in welcher

$R^1$ und X die oben angegebene Bedeutung haben, mit Iso(thio)cyanaten der Formel (IV),

$R^2$-N = C = Y (IV)

in welcher

$R^2$ und Y die oben angegebene Bedeutung haben,

gegebenenfalls in Gegenwart eines Verdünnungsmittels und gegebenenfalls in Gegenwart eines Reaktionshilfsmittels umsetzt,

oder wenn man

c) Triazolinone der Formel (V),

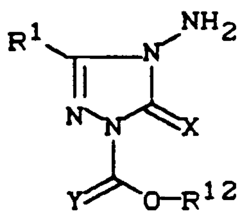

(V)

in welcher

$R^1$, X und Y die oben angegebene Bedeutung haben und

$R^{12}$ für Alkyl, Aralkyl oder Aryl steht,

mit Aminen der Formel (VI),

$R^2$-$NH_2$ (VI)

in welcher

$R^2$ die oben angegebene Bedeutung hat,

gegebenenfalls in Gegenwart eines Verdünnungsmittels und gegebenenfalls in Gegenwart

eines Reaktionshilfsmittels umsetzt.

Schließlich wurde gefunden, daß die neuen substituierten Triazolinone der allgemeinen Formel (I) herbizide Eigenschaften besitzen.

Überraschenderweise zeigen die erfindungsgemäßen substituierten Triazolinone der allgemeinen Formel (I) eine erheblich bessere herbizide Wirksamkeit gegenüber Problemunkräutern und gleichzeitig eine vergleichbar gute Verträglichkeit gegenüber wichtigen Kulturpflanzen im Vergleich zu den aus dem Stand der Technik bekannten substituierten Triazolinonen, wie beispielsweise die Verbindung 1-(4-Phenyl-2-butylaminocarbonyl)-3-methyl-4-amino-1,2,4-triazolin-5-on, welche chemisch und wirkungsmäßig naheliegende Verbindungen sind.

Die erfindungsgemäßen substituierten Triazolinone sind durch die Formel (I) allgemein definiert. Bevorzugt sind Verbindungen der Formel (I), bei welchen

$R^1$ für geradkettiges oder verzweigtes Alkyl mit 1 bis 6 Kohlenstoffatomen oder für Cycloalkyl mit 3 bis 7 Kohlenstoffatomen steht,

$R^2$ für gegebenenfalls einfach oder mehrfach, gleich oder verschieden substituiertes Cycloalkyl mit 3 bis 7 Kohlenstoffatomen steht, wobei als Substituenten infrage kommen:
im Arylteil jeweils gegebenenfalls einfach oder mehrfach, gleich oder verschieden substituiertes Aryl, Arylalkyl, Arylalkenyl oder Arylalkinyl mit jeweils 6 oder 10 Kohlenstoffatomen im Arylteil und gegebenenfalls bis zu 6 Kohlenstoffatomen in den jeweiligen geradkettigen oder verzweigten Alkyl-bzw. Alkenyl- oder Alkinylteilen, wobei als Arylsubstituenten jeweils infrage kommen:
Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl,

$R^2$ außerdem für einen Rest der Formel

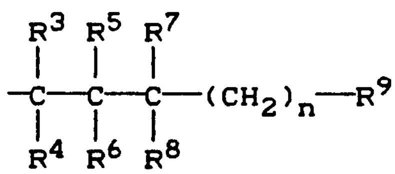

steht,

X für Sauerstoff oder Schwefel steht und

Y für Sauerstoff oder Schwefel steht, wobei

$R^3$ für geradkettiges oder verzweigtes Alkyl mit 1 bis 8 Kohlenstoffatomen steht,

$R^4$, $R^5$, $R^6$, $R^7$ und $R^8$ unabhängig voneinander jeweils für Wasserstoff oder für geradkettiges oder verzweigtes Alkyl mit 1 bis 4 Kohlenstoffatomen stehen,

$R^9$ für gegebenenfalls einfach oder mehrfach, gleich oder verschieden substituiertes Cycloalkyl mit 3 bis 7 Kohlenstoffatomen steht, wobei als Substituenten infrage kommen:
Halogen, Cyano, jeweils geradkettiges oder verzweigtes Alkyl mit 1 bis 4 Kohlenstoffatomen sowie jeweils geradkettiges oder verzweigtes Halogenalkyl mit jeweils 1 bis 4 Kohlenstoffatomen und 1 bis 9 gleichen oder verschiedenen Halogenatomen;
außerdem für jeweils gegebenenfalls einfach oder mehrfach, gleich oder verschieden substituiertes Aryl mit 6 bis 10 Kohlenstoffatomen oder Heteroaryl mit 2 bis 9 Kohlenstoffatomen und 1 bis 4 gleichen oder verschiedenen Heteroatomen - insbesondere Stickstoff, Sauerstoff und/oder Schwefel - steht, wobei als Substituenten jeweils infrage kommen: Halogen, Cyano, Nitro, jeweils geradkettiges oder verzweigtes Alkyl, Alkoxy oder Alkylthio mit jeweils 1

bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen; zweifach verknüpftes, geradkettiges oder verzweigtes, gegebenenfalls ein-bis vierfach, gleich oder verschieden durch Halogen substituiertes Dioxyalkylen mit 1 bis 3 Kohlenstoffatomen; Dialkylamino, N-Alkanoylamino, Alkoxycarbonyl oder Alkoximinoalkyl mit jeweils 1 bis 4 Kohlenstoffatomen in den einzelnen geradkettigen oder verzweigten Alkylteilen sowie jeweils gegebenenfalls einfach oder mehrfach, gleich oder verschieden durch Halogen und/oder geradkettiges oder verzweigtes Alkyl mit 1 bis 4 Kohlenstoffatomen substituiertes Aryl oder Aryloxy mit 6 oder 10 Kohlenstoffatomen und

n für eine Zahl 0, 1, 2 oder 3 steht;

wobei jedoch Verbindungen, bei denen gleichzeitig $R^1$ für einen Methylrest, X für Sauerstoff, Y für Sauerstoff und $R^2$ für einen Rest der Formel

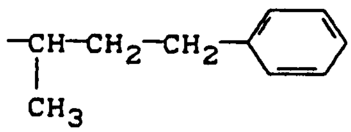 ; 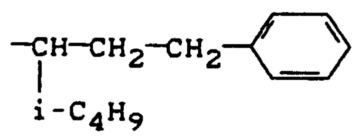 ;

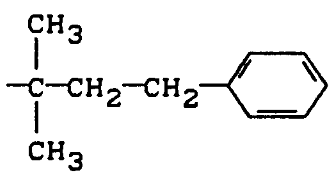 oder 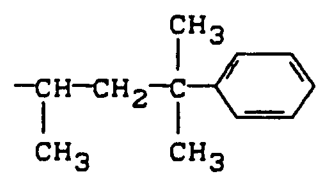

steht, ausgenommen sind.

Besonders bevorzugt sind Verbindungen der Formel (I), bei welchen

$R^1$ für Methyl, Ethyl, n- oder i-Propyl, n-, i-, s- oder t-Butyl, Cyclopropyl, Cyclobutyl, Cyclopentyl oder Cyclohexyl steht,

$R^2$ für jeweils gegebenenfalls ein-bis zweifach, gleich oder verschieden substituiertes Cyclopropyl, Cyclobutyl, Cyclopentyl oder Cyclohexyl steht, wobei als Substituenten jeweils infrage kommen:
im Arylteil jeweils gegebenenfalls ein-bis dreifach, gleich oder verschieden substituiertes Phenyl, Benzyl, Phenylethyl, Phenylpropyl, Phenylethenyl, Phenylpropenyl, Phenylethinyl, Phenylpropinyl, α-Naphthyl oder β-Naphthyl, wobei als Arylsubstituenten jeweils infrage kommen:
Fluor, Chlor, Brom, Cyano, Nitro, Methyl, Ethyl, n- oder i-Propyl , n-, i-, s- oder t-Butyl, Methoxy, Ethoxy, n- oder i-Propoxy, n-, i-, s- oder t-Butoxy, Methylthio, Ethylthio, Trifluormethyl, Trifluormethoxy, Trifluormethylthio, Methoxycarbonyl, Ethoxycarbonyl, Methoximinomethyl, Methoximinoethyl, Ethoximinomethyl, Ethoximinoethyl oder gegebenenfalls ein- bis dreifach, gleich oder verschieden durch Fluor, Chlor, Brom, Methyl und/oder Ethyl substituiertes Phenyl,

$R^2$ außerdem für einen Rest der Formel

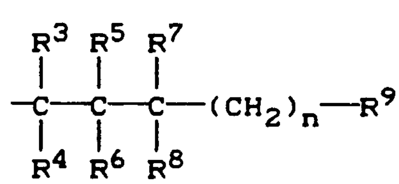

steht,

X für Sauerstoff oder Schwefel steht und

Y für Sauerstoff oder Schwefel steht, wobei

$R^3$ für geradkettiges oder verzweigtes Alkyl mit 1 bis 6 Kohlenstoffatomen steht,

$R^4$, $R^5$, $R^6$, $R^7$ und $R^8$ für Wasserstoff oder für geradkettiges oder verzweigtes Alkyl mit 1 bis 3 Kohlenstoffatomen stehen,

$R^9$ für jeweils gegebenenfalls ein-bis fünffach, gleich oder verschieden substituiertes Cyclopropyl, Cyclobutyl, Cyclopentyl oder Cyclohexyl steht, wobei als Substituenten jeweils infrage kommen:

Fluor, Chlor, Brom, Cyano, Methyl, Ethyl, n-oder i-Propyl, Chlormethyl, Dichlormethyl, Trichlormethyl, Difluormethyl oder Trifluormethyl;

außerdem für jeweils gegebenenfalls ein-bis dreifach, gleich oder verschieden substituiertes Phenyl, α-Naphthyl, β-Naphthyl, Pyridyl, Pyrimidyl, Triazinyl, Pyridazinyl, Pyrazolyl, Imidazolyl, Triazolyl, Thiazolyl, Isothiazolyl, Benzthiazolyl, Oxazolyl, Isoxazolyl, Benzoxazolyl, Benzopyrazolyl, Pyrrolyl, Furanyl, Thienyl, Indoyl, Benzopyranyl oder Chinolyl steht, wobei als Substituenten jeweils infrage kommen:

Fluor, Chlor, Brom, Cyano, Nitro, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, Difluormethyl, Trifluormethyl, Difluormethoxy, Trifluormethoxy, Difluormethylthio, Trifluormethylthio, Dimethylamino, Diethylamino, N-Acetamido, Dioxymethylen, Difluordioxymethylen, Dioxyethylen, Trifluordioxyethylen, Tetrafluordioxyethylen, Methoxycarbonyl, Ethoxycarbonyl, Propoxycarbonyl, Methoximinomethyl, Methoximinoethyl, Ethoximinomethyl, Ethoximinoethyl oder gegebenenfalls ein- bis dreifach, gleich oder verschieden durch Fluor, Chlor, Brom, Methyl, Ethyl, Methoxy und/oder Ethoxy substituiertes Phenyl, Phenoxy, α-Naphthyl oder β-Naphthyl und

n für eine Zahl 0, 1 oder 2 steht,

wobei jedoch Verbindungen, bei denen gleichzeitig $R^1$ für einen Methylrest, X für Sauerstoff, Y für Sauerstoff und $R^2$ für einen Rest der Formel

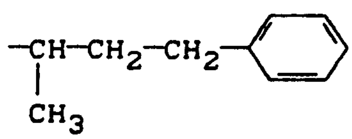 ; 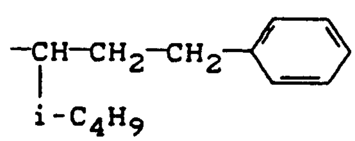 ;

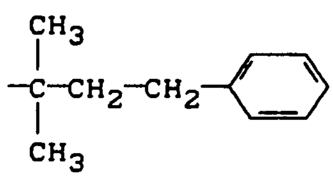 oder 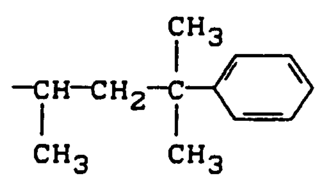

steht, ausgenommen sind.

Ganz besonders bevorzugt sind Verbindungen der Formel (I), bei welchen

$R^1$ für Methyl, Ethyl, n- oder i-Propyl oder für Cyclopropyl steht,

$R^2$ für einen Rest der Formel

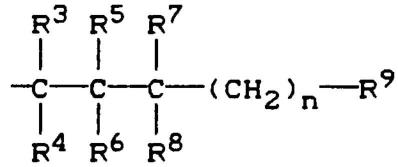

steht,

X für Sauerstoff steht,

Y für Sauerstoff steht,

$R^3$ für Methyl, Ethyl, n- oder i-Propyl, n-, i-, s- oder t-Butyl steht,

$R^4$, $R^5$, $R^6$, $R^7$ und $R^8$ unabhängig voneinander jeweils für Wasserstoff, Methyl oder Ethyl stehen,

$R^9$ für gegebenenfalls ein- bis dreifach, gleich oder verschieden substituiertes Phenyl steht, wobei als Substituenten jeweils infrage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, Difluormethyl, Trifluormethyl, Difluormethoxy, Trifluormethoxy, Difluormethylthio, Trifluormethylthio, Dimethylamino, Diethylamino, N-Acetamido, Dioxymethylen, Difluordioxymethylen, Dioxyethylen, Trifluordioxyethylen, Tetrafluordioxyethylen, Methoxycarbonyl, Ethoxycarbonyl, Propoxycarbonyl, Methoximinomethyl, Methoximinoethyl, Ethoximinomethyl, Ethoximinoethyl oder jeweils gegebenenfalls ein- bis dreifach, gleich oder verschieden durch Fluor, Chlor, Brom, Methyl, Ethyl, Methoxy und/oder Ethoxy substituiertes Phenyl, Phenoxy, α-Naphthyl oder β-Naphthyl und

n für eine Zahl 0 oder 1 steht,

wobei jedoch Verbindungen, bei denen gleichzeitig $R^1$ für einen Methylrest, X für Sauerstoff, Y für Sauerstoff und $R^2$ für einen Rest der Formel

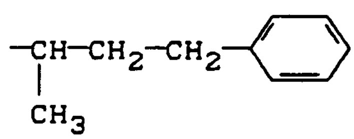 ; 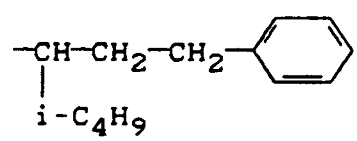 ;

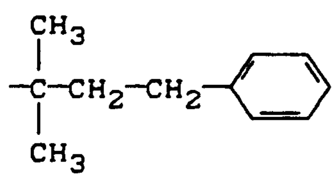 oder 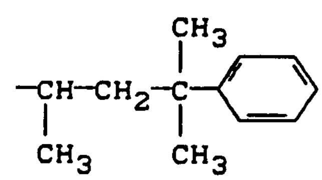

steht, ausgenommen sind.

Im einzelnen seien die bei den Herstellungsbeispielen aufgeführten Verbindungen genannt.

Verwendet man beispielsweise 1-{N-[4-(4-Chlorphenyl)-2-methyl-but-2-yl]-carbamoyl}-4-isopropylidenimino-3-methyl-1,2,4-triazolin-5-on als Ausgangsverbindung, so läßt sich der Reaktionsablauf des erfindungsgemäßen Verfahrens (a) durch das folgende Formelschema darstellen:

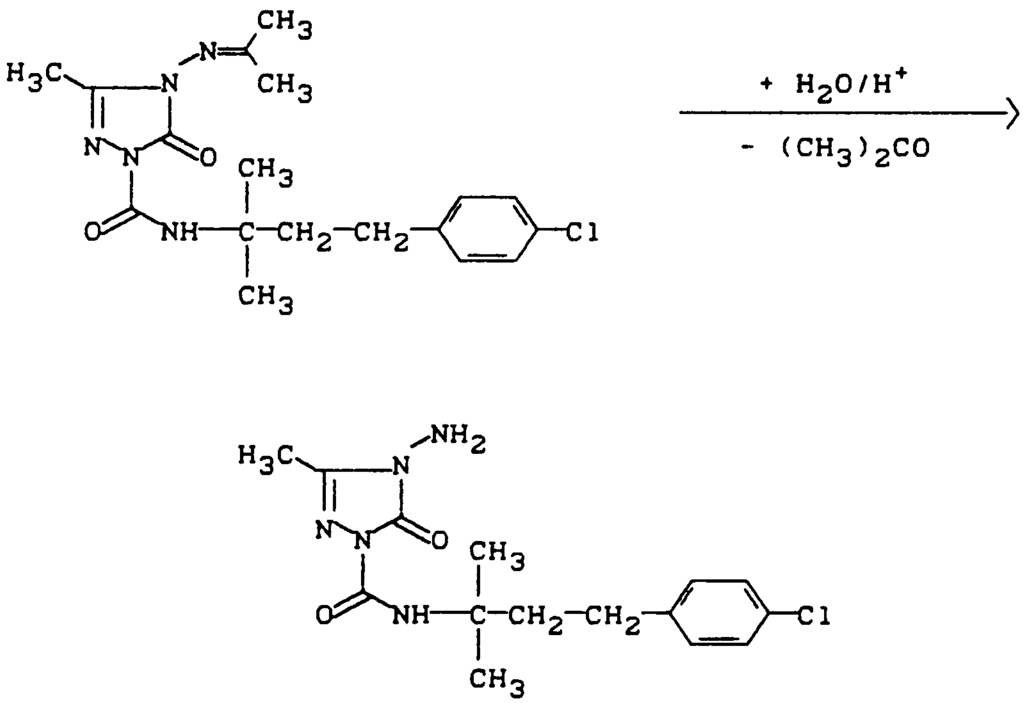


Verwendet man beispielsweise 4-Amino-3-methyl-1,2,4-(1H)-triazolin-5-on und 3-Methyl-4-phenyl-but-2-yl-isocyanat als Ausgangsstoffe, so läßt sich der Reaktionsablauf des erfindungsgemäßen Verfahrens (b) durch das folgende Formelschema darstellen:

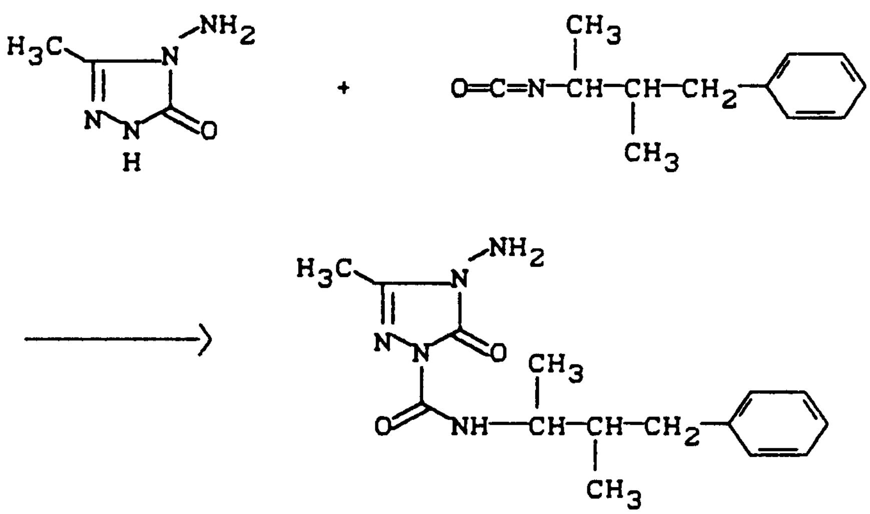

Verwendet man beispielsweise 1-Ethoxycarbonyl-4-amino-3-methyl-1,2,4-triazolin-5-on und 4-Phenyl-2-ethyl-but-2-ylamin als Ausgangsstoffe, so läßt sich der Reaktionsablauf des erfindungsgemäßen Verfahrens (c) durch das folgende Formelschema darstellen:

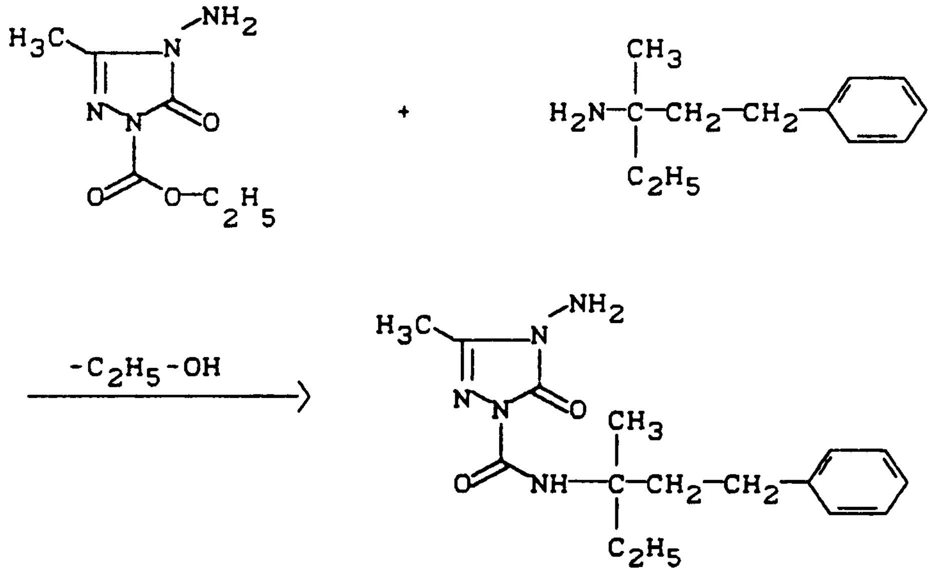


Die zur Durchführung des erfindungsgemäßen Verfahrens (a) als Ausgangsstoffe benötigten Hydrazone sind durch die Formel (II) allgemein definiert. In dieser Formel (II) stehen $R^1$, $R^2$, X und Y vorzugsweise für diejenigen Reste, die bereits im Zusammenhang mit der Beschreibung der erfindungsgemäßen Verbindungen der Formel (I) als bevorzugt für diese Substituenten genannt wurden.
$R^{10}$ und $R^{11}$ stehen vorzugsweise jeweils unabhängig voneinander für Wasserstoff, für geradkettiges oder verzweigtes Alkyl mit 1 bis 4 Kohlenstoffatomen, für Phenyl oder für Benzyl.
Die Hydrazone der Formel (II) sind noch nicht bekannt. Sie sind ebenfalls Gegenstand der vorliegenden Erfindung. Man erhält sie in Analogie zu bekannten Verfahren (vergleiche z. B. DE-OS 38 03 523 oder Acta. Pol. Pharm. 38, 153-162 [1981] bzw. CA 95: 203841), beispielsweise wenn man 1H-Triazolinone der Formel (III),

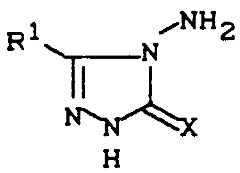

(III)

in welcher
$R^1$ und X die oben angegebene Bedeutung haben,
mit Aldehyden oder Ketonen der Formel (VII),

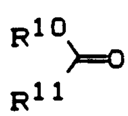

(VII)

in welcher
$R^{10}$ und $R^{11}$ die oben angegebene Bedeutung haben,
gegebenenfalls in Gegenwart eines Verdünnungsmittels wie beispielsweise Dichlormethan oder Toluol und gegebenenfalls in Gegenwart eines Reaktionshilfsmittels wie beispielsweise p-Toluolsulfonsäure bei Temperaturen zwischen 40 ° C und 120 ° C umsetzt und die so erhältlichen 1-unsubstituierten Triazolinon-Hydrazone der Formel (VIII),

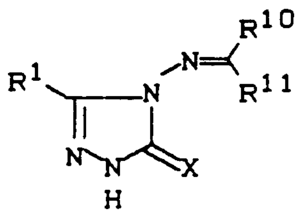

(VIII)

in welcher
$R^1$, $R^{10}$, $R^{11}$ und X die oben angegebene Bedeutung haben,
entweder in einer anschließenden 2. Stufe mit Iso(thio)cyanaten der Formel (IV),

$R^2$-N = C = Y (IV)

in welcher
$R^2$ und Y die oben angegebene Bedeutung haben,
gegebenenfalls in Gegenwart eines Verdünnungsmittels wie beispielsweise Dichlormethan oder Dioxan und gegebenenfalls in Gegenwart eines Reaktionshilfsmittels wie beispielsweise Triethylamin bei Temperaturen zwischen 0 °C und 150 °C umsetzt oder alternativ in einer anschließenden 2. Stufe mit (Thio-)Chlorameisensäureestern der Formel (IX),

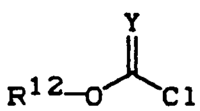

(IX)

in welcher
$R^{12}$ für Alkyl, Aralkyl oder Aryl steht und
Y die oben angegebene Bedeutung hat,
oder mit (Thio-)Phosgen gegebenenfalls in Gegenwart eines Verdünnungsmittels wie beispielsweise Chloroform, Dichlormethan oder Tetrahydrofuran und gegebenenfalls in Gegenwart eines Reaktionshilfsmittels wie beispielsweise Triethylamin, Natriumhydrid oder Kalium-tert-butylat bei Temperaturen zwischen -20 °C und +100 °C umsetzt und die so erhältlichen Triazolinon-Derivate der Formel (X),

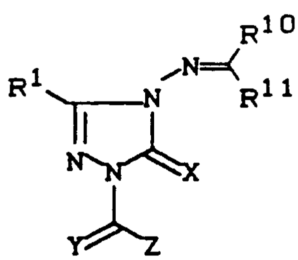

(X)

in welcher
Z für einen Rest -O-$R^{12}$ oder für Chlor steht und
$R^1$, $R^{10}$, $R^{11}$, X und Y die oben angegebene Bedeutung haben,
in einer anschließenden 3. Stufe mit Aminen der Formel (VI),

$R^2$-$NH_2$ (VI)

in welcher
$R^2$ die oben angegebene Bedeutung hat,
gegebenenfalls in Gegenwart eines Verdünnungsmittels wie beispielsweise Tetrahydrofuran und gegebenenfalls in Gegenwart eines Reaktionshilfsmittels wie beispielsweise Natriumhydroxid oder Kaliumhydroxid

bei Temperaturen zwischen 20 ° C und 50 ° C umsetzt.

Triazolinon-Derivate der Formel (X) erhält man alternativ auch, wenn man Triazolinone der Formel (V),

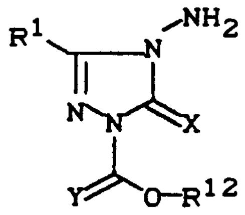

(V)

in welcher

$R^1$, X und Y die oben angegebene Bedeutung haben und

$R^{12}$ für Alkyl, Aralkyl oder Aryl steht,

mit Aldehyden oder Ketonen der Formel (VII),

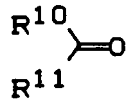

(VII)

in welcher

$R^{10}$ und $R^{11}$ die oben angegebene Bedeutung haben,

gegebenenfalls in Gegenwart eines Verdünnungsmittels wie beispielsweise Dichlormethan oder Toluol und gegebenenfalls in Gegenwart eines Reaktionshilfsmittels wie beispielsweise p-Toluolsulfonsäure bei Temperaturen zwischen 40 ° C und 120 ° C umsetzt.

Die zur Durchführung des erfindungsgemäßen Verfahrens (b) und zur Synthese der Vorprodukte der Formel (II) als Ausgangsprodukte erforderlichen 1H-Triazolinone sind durch die Formel (III) allgemein definiert. In dieser Formel (III) stehen $R^1$ und X vorzugsweise für diejenigen Reste, die bereits im Zusammenhang mit der Beschreibung der erfindungsgemäßen Stoffe der Formel (I) als bevorzugt für diese Substituenten genannt wurden.

Die 1H-Triazolinone der Formel (III) sind bekannt oder erhältlich in Analogie zu bekannten Verfahren (vergleiche z. B. J. Heterocycl. Chem. 16, 403 [1979]; J. Heterocycl` Chem. 17, 1691 [1980]; Europ. J. Med. Chem. 18, 215 [1983]; Chem. Ber. 98, 3025 [1965]; Liebigs Ann. Chem, 637, 135 [1960]; DE-OS 37 19 575; DE-OS 38 03 523).

Die zur Durchführung des erfindungsgemäßen Verfahrens (b) und gegebenenfalls zur Synthese der Vorprodukte der Formel (II) weiterhin als Ausgangsprodukte erforderlichen Iso(thio)cyanate sind durch die Formel (IV) allgemein definiert. In dieser Formel (IV) stehen $R^2$ und Y vorzugsweise für diejenigen Reste, die bereits im Zusammenhang mit der Beschreibung der erfindungsgemäßen Stoffe der Formel (I) als bevorzugt für diese Substituenten genannt wurden.

Die Iso(thio)cyanate der Formel (IV) sind teilweise bekannt (vergleiche z. B. Nouv. J. Chim. 1, 243-254 [1977] bzw. CA 87: 151614a; US 4.035.404) oder erhältlich in Analogie zu bekannten Verfahren (vergleiche z. B. Synthesis 1977, 756; Org. Syntheses Coll. Vol. IV, 521 [1963] oder "Organikum" VEB Deutscher Verlag der Wissenschaften Berlin 1981, S. 703) beispielsweise, wenn man Amine der Formel (VI),

$R^2$-$NH_2$ (VI)

in welcher

$R^2$ die oben angegebene Bedeutung hat,

mit Phosgen oder Thiophosgen gegebenenfalls in Gegenwart eines Verdünnungsmittels wie beispielsweise Chloroform, Toluol, Chlorbenzol oder Tetrahydrofuran und gegebenenfalls in Gegenwart eines Reaktionshilfsmittels wie beispielsweise Triethylamin, Natriumhydroxid oder Kaliumhydroxid bei Temperaturen zwischen -20 ° C und +150 ° C umsetzt.

Die zur Durchführung des erfindungsgemäßen Verfahrens (c) und gegebenenfalls zur Synthese der Vorprodukte der Formel (II) weiterhin als Ausgangsprodukte erforderlichen Triazolinone sind durch die Formel (V) allgemein definiert. In dieser Formel (V) stehen $R^1$, X und Y vorzugsweise für diejenigen Reste,

die bereits im Zusammenhang mit der Beschreibung der erfindungsgemäßen Stoffe der Formel (I) als bevorzugt für diese Substituenten genannt wurden.

$R^{12}$ steht vorzugsweise für geradkettiges oder verzweigtes Alkyl mit 1 bis 4 Kohlenstoffatomen, für Phenyl oder für Benzyl.

Die Triazolinone der Formel (V) sind bekannt oder erhältlich in Analogie zu bekannten Verfahren (vergleiche z. B. DE-OS 37 19 575; DE-OS 38 03 523).

Die zur Durchführung des erfindungsgemäßen Verfahrens (c) und gegebenenfalls zur Synthese der Vorprodukte der Formeln (II) und (IV) weiterhin als Ausgangsstoffe benötigten Amine sind durch die Formel (VI) allgemein definiert. In dieser Formel (VI) steht $R^2$ vorzugsweise für diejenigen Reste, die bereits im Zusammenhang mit der Beschreibung der erfindungsgemäßen Verbindungen der Formel (I) als bevorzugt für diesen Substituenten genannt wurden.

Die Amine der Formel (VI) sind bekannt oder erhältlich in Analogie zu bekannten Verfahren (vergleiche z. B. Coll. Czech. Chem. Commun. 35, 2810-2830 [1970]; Synth. Commun. 18, 29-35 [19881; Angew. Chem. 96, 368-369 [1984]; DE-OS 28 25 961; DE-OS 32 22 152; US 4.906.645; EP 320 898; US 4.695.589; EP 237 305; US 4.374.149; EP 28 105; EP 6 614; Nouv. J. Chim. 1, 243-254 [1977] bzw. CA 87: 151614a).

Aldehyde und Ketone der Formel (VII) sind allgemein bekannte Verbindungen der organischen Chemie.

(Thio-)Chlorameisensäureester der Formel (IX) sind ebenfalls allgemein bekannte Verbindungen der organischen Chemie.

Als Säuren zur Durchführung des erfindungsgemäßen Verfahrens (a) kommen alle üblicherweise für Hydrazonspaltungen verwendbaren anorganischen oder organischen Säuren infrage. Vorzugsweise verwendet man anorganische Mineralsäuren wie Chlorwasserstoffsäure, Schwefelsäure oder Phosphorsäure.

Als Verdünnungsmittel zur Durchführung des erfindungsgemäßen Verfahrens (a) kommen alle üblichen organischen oder anorganischen Lösungsmittel infrage. Vorzugsweise verwendet man polare mit Wasser mischbare organische Lösungsmittel, insbesondere Alkohole, wie Methanol, Ethanol, Propanol oder Butanol, deren Gemische mit Wasser oder reines Wasser als Lösungsmittel.

Die Reaktionstemperaturen können bei der Durchführung des erfindungsgemäßen Verfahrens (a) in einem größeren Bereich variiert werden. Im allgemeinen arbeitet man bei Temperaturen zwischen +20 °C und +150 °C, vorzugsweise bei Temperaturen zwischen +50 °C und +120 °C.

Das erfindungsgemäße Verfahren (a) wird üblicherweise unter Normaldruck oder unter vermindertem Druck durchgeführt. Arbeitet man unter vermindertem Druck, so kommen Druckbereiche zwischen 20 und 400 mbar, vorzugsweise zwischen 100 und 200 mbar infrage.

Zur Durchführung des erfindungsgemäßen Verfahrens (a) setzt man pro Mol an Hydrazon der Formel (II) im allgemeinen 1,0 bis 5,0 Mol, vorzugsweise 1,0 bis 2,5 Mol an Säure ein. Dabei löst man das Hydrazon der Formel (II) in einer geeigneten Menge an Verdünnungsmittel, setzt dann die erforderliche Menge Säure zu und engt die Mischung unter vermindertem Druck über mehrere Stunden langsam ein.

In einer besonderen Durchführungsform ist es auch möglich, das erfindungsgemäße Verfahren (a) und die Herstellung der dafür erforderlichen Vorprodukte der Formel (II) in einem Reaktionsschritt in einem sogenannten "Eintopfverfahren" durchzuführen. Dabei geht man so vor, daß man als Ausgangsverbindungen entweder die Triazolinon-Derivate der Formel (X) wählt und diese nacheinander im "Eintopfverfahren" mit Aminen der Formel (VI) und anschließend mit Säure gemäß dem erfindungsgemäßen Verfahren (a) umsetzt oder alternativ, daß man als Ausgangsverbindungen die 1-unsubstituierten Triazolin-Hydrazone der Formel (VIII) wählt und diese nacheinander im "Eintopfverfahren" mit Iso(thio)cyanaten der Formel (IV) und anschließend mit Säure gemäß dem erfindungsgemäßen Verfahren (a) umsetzt.

Die Reaktionsdurchführung, Aufarbeitung und Isolierung der Reaktionsprodukte erfolgt nach bekannten Verfahren (vergleiche hierzu beispielsweise DE-OS 38 03 523 oder die Herstellungsbeispiele).

Als Verdünnungsmittel zur Durchführung des erfindungsgemäßen Verfahrens (b) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran oder Ethylenglykoldimethyl- oder -diethylether; Ketone, wie Aceton, Butanon oder Methyl-isobutyl-keton; Nitrile, wie Acetonitril, Propionitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester oder Sulfoxide, wie Dimethylsulfoxid.

Das erfindungsgemäße Verfahren (b) kann gegebenenfalls in Gegenwart eines geeigneten Reaktionshilfsmittels durchgeführt werden. Als solche kommen alle üblichen anorganischen oder organischen Basen infrage.

Vorzugsweise verwendet man tertiäre Amine, wie Trimethylamin, Triethylamin, Tributylamin, N,N-Dimethylanilin, Pyridin, Piperidin, N-Methylpiperidin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (b) in einem größeren Bereich variiert werden. Im allgemeinen arbeitet man bei Temperaturen zwischen 0 °C und +150 °C, vorzugsweise bei Temperaturen zwischen +10 °C und +100 °C.

Zur Durchführung des erfindungsgemäßen Verfahrens (b) setzt man pro Mol an 1H-Triazolinon der Formel (III) im allgemeinen 1,0 bis 2,0 Mol, vorzugsweise 1,0 bis 1,5 Mol an Iso(thio)cyanat der Formel (IV) und gegebenenfalls 0,001 bis 2,0 Mol, vorzugsweise 0,001 bis 1,0 Mol an Reaktionshilfsmittel ein.
Die Reaktionsdurchführung, Aufarbeitung und Isolierung der Reaktionsprodukte erfolgt nach bekannten Verfahren (vergleiche hierzu beispielsweise DE-OS 38 03 523 oder die Herstellungsbeispiele.

Als Verdünnungsmittel zur Durchführung des erfindungsgemäßen Verfahrens (c) kommen ebenfalls inerte organische Lösungsmittel infrage. Vorzugsweise verwendet man die bei Verfahren (b) genannten Verdünnungsmittel.

Das erfindungsgemäße Verfahren (c) kann gegebenenfalls in Gegenwart eines geeigneten basischen Reaktionshilfsmittels durchgeführt werden. Als solche kommen alle üblichen anorganischen oder organischen Basen infrage.

Hierzu gehören beispielsweise Erdalkali- oder Alkalimetallhydroxide, wie Natriumhydroxid, Calciumhydroxid, Kaliumhydroxid oder auch Ammoniumhydroxid, Alkalimetallcarbonate, wie Natriumcarbonat, Kaliumcarbonat, Kaliumhydrogencarbonat, Natriumhydrogencarbonat oder Ammoniumcarbonat, Alkali- oder Erdalkalimetallacetate, wie Natriumacetat, Kaliumacetat, Calciumacetat oder Ammoniumacetat, sowie tertiäre Amine, wie Trimethylamin, Triethylamin, Tributylamin, N,N-Dimethylanilin, Pyridin, Piperidin, N-Methylpiperidin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (c) in einem größeren Bereich variiert werden. Im allgemeinen arbeitet man bei Temperaturen zwischen 0 °C und +120 °C, vorzugsweise bei Temperaturen zwischen +20 °C und +50 °C.

Das erfindungsgemäße Verfahren (c) wird üblicherweise unter Normaldruck durchgeführt.

Zur Durchführung des erfindungsgemäßen Verfahrens (c) setzt man pro Mol Triazolinon der Formel (V) im allgemeinen 1,0 bis 5,0 Mol, vorzugsweise 1,0 bis 2,5 Mol Base als Reaktionshilfsmittel ein.
Die Reaktionsdurchführung, Aufarbeitung und Isolierung der Reaktionsprodukte erfolgt nach bekannten Verfahren (vergleiche hierzu beispielsweise DE-OS 38 03 523 oder die Herstellungsbeispiele).

Die Reinigung der Endprodukte der Formel (I) erfolgt mit Hilfe üblicher Verfahren, beispielsweise durch Säulenchromatographie oder durch Umkristallisieren.
Die Charakterisierung erfolgt mit Hilfe des Schmelzpunktes oder bei nicht kristallisierenden Verbindungen mit Hilfe der Protonen-Kernresonanzspektroskopie ($^1$H-NMR).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:
Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.
Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und

Sportrasen und Weideflächen und zur selektiven Unkrautbekämpfung in einjährigen Kulturen eingesetzt werden.

Dabei lassen sich die erfindungsgemäßen Wirkstoffe mit besonders gutem Erfolg zur Bekämpfung von mono- und dikotylen Unkräutern in monokotylen und dikotylen Kulturen wie beispielsweise Weizen, Mais, Soja, Baumwolle oder Raps einsetzen.

Daneben greifen die erfindungsgemäßen Wirkstoffe auch in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid oder Dimethylsulfoxid, sowie Wasser. Als feste Trägerstoffe kommen infrage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen infrage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N'-dimethyl-harnstoff (METABENZTHIAZURON) zur Unkrautbekämpfung in Getreide; 4-Amino-3-methyl-6-phenyl-1,2,4-triazin-5-

(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, infrage. Auch Mischungen mit 2,4-Dichlorphenoxyessigsäure (2,4-D); 4-(2,4-Dichlorphenoxy)-buttersäure (2,4-DB); 2,4-Dichlorphenoxypropionsäure (2,4-DP); (2-Methyl-4-chlorphenoxy)-essigsäure (MCPA); (4-Chlor-2-methylphenoxy)-propionsäure (MCPP); [(4-Amino-3,5-dichlor-6-fluor-2-pyridinyl)-oxy]-essigsäure bzw, deren 1-Methylheptylester (FLUROXYPYR); 2-[4-(2,4-Dichlorphenoxy)-phenoxyl-propionsäure, deren Methyl- oder deren Ethylester (DICLOFOP); 2-{4-[(6-Chlor-2-benzoxazolyl}-oxy]-phenoxy}-propansäure, deren Methyl- oder deren Ethylester (FENOXAPROP); 2-[4-(5-Trifluormethyl-2-pyridyloxy)-phenoxy]-propansäure oder deren Butylester (FLUAZIFOP); 2-[4-(6-Chlor-chinoxalin-2-yl-oxy)-phenoxy]-propionsäure-ethylester (QUIZALOFOPETHYL); 2-{4-[(3-Chlor-5-(trifluormethyl)-2-pyridinyl)-oxy]-phenoxy}-propansäure bzw. deren Ethylester (HALOXYFOP); 3,6-Dichlor-2-pyridincarbonsäure (CLOPYRALID); 5-Amino-4-chlor-2-phenyl-2,3-dihydro-3-oxy-pyridazin (CHLORIDAZON); 3-(Ethoxycarbonylaminophenyl)-N-(3'-methylphenyl)-carbamat (PHENMEDIPHAM); Isopropyl-N-phenyl-carbamat (PROPHAM); N-(3-Chlorphenyl)-isopropylcarbamat (CHLORPROPHAM); 4-Amino-benzolsulfonyl-methylcarbamat (ASULAM); Chloressigsäure-N-(methoxymethyl)-2,6-diethylanilid (ALACHLOR); N-(Butoxymethyl)-2-chlor-N-(2,6-diethylphenyl)-acetamid (BUTACHLOR); 2-Chlor-N-(2,6-dimethylphenyl)-N-[(1H)-pyrazol-1-yl-methyl]-acetamid (METAZACHLOR); 2-Ethyl-6-methyl-N-(1-methyl-2-methoxyethyl)-chloracetanilid (METOLACHLOR); 4-(Di-n-propylamino)-3,5-dinitrobenzolsulfonamid (ORYZALIN); N-(1-Ethylpropyl)-3,4-dimethyl-2,6-dinitroanilin (PENDIMETHALIN); 2,6-Dinitro-4-trifluormethyl-N,N-dipropylanilin (TRIFLURALIN); 5-(2-Chlor-4-trifluormethyl-phenoxy)-2-nitro-benzoesäure (ACIFLUORFEN); Methyl-5-(2,4-dichlorphenoxy)-2-nitrobenzoat (BIFENOX); 5-(2-Chlor-4-trifluormethyl-phenoxy)-N-methylsulfonyl-2-nitrobenzamid (FOMESAFEN); (2-Ethoxy-1-methyl-2-oxo-ethyl)-5-[2-chlor-4-(trifluormethyl)-phenoxy]-2-nitrobenzoat (LACTOFEN); (2-Chlor-4-trifluormethylphenyl)-(3-ethoxy-4-nitro-phenyl)-ether (OXYFLUORFEN); N,N-Dimethyl-N'-(3-chlor-4-methylphenyl)-harnstoff (CHLORTOLURON); N,N-Dimethyl-N'-(3-trifluormethylphenyl)-harnstoff (FLUOMETURON); N,N-Dimethyl-N'-(4-isopropylphenyl)-harnstoff (ISOPROTURON); Methyl-6,6-dimethyl-2,4-dioxo-3-[1-(2-propenyloxyamino)-butyliden]-cyclohexancarbonsäure (ALLOXYDIM); 2-[1-(Ethoximino)-butyl]-3-hydroxy-5-[tetrahydro-(2H)-thiopyran-3-yl]-2-cyclohexen-1-on (CYCLOXYDIM); 2-[1-(Ethoxamino)-butyliden]-5-(2-ethylthiopropyl)-1,3-cyclohexadion (SETHOXYDIM); 2-[4,5-Dihydro-4-methyl-4-isopropyl-5-oxo-(1H)-imidazol-2-yl]-5-ethyl-pyridin-3-carbonsäure (IMAZETHAPYR); Methyl2-[4,5-dihydro-4-methyl-4-(1-methylethyl)-5-oxo-1H-imidazol-2-yl]-4(5)-methylbenzoat (IMAZAMETHABENZ); 2-[5-Methyl-5-(1-methylethyl)-4-oxo-2-imidazolin-2-yl]-3-chinolincarbonsäure (IMAZAQUIN); 3,5-Dibrom-4-hydroxy-benzonitril (BROMOXYNIL); 3,5-Diiod-4-hydroxybenzonitril (IOXYNIL); N-Methyl-2-(1,3-benzthiazol-2-yloxy)-acetanilid (MEFENACET); Ethyl-2-{[(4-chlor-6-methoxy-2-pyrimidinyl)-aminocarbonyl]-aminosulfonyl}-benzoat (CHLORIMURON); 2-Chlor-N-{[(4-methoxy-6-methyl-1,3,5-triazin-2-yl)-amino]-carbonyl}-benzolsulfonamid (CHLORSULFURON); 2-{[[((4-Methoxy-6-methyl-1,3,5-triazin-2-yl)-amino)-carbonyl]-amino]-sulfonyl}-benzoesäure oder deren Methylester (METSULFURON); 3-[[[[(4-Methoxy-6-methyl-1,3,5-triazin-2-yl)-amino]-carbonyl]-amino]-sulfonyl]-thiophen-2-carbonsäure-methylester (THIAMETURON); N,S-Diethyl-N-cyclohexyl-thiolcarbamat (CYCLOATE); N,N-Di-n-propyl-thiocarbamidsäure-S-ethylester (EPTAME); N,N-Diisopropyl-S-(2,3,3-trichlorallyl)-thiolcarbamat (TRIALLATE); 2-Chlor-4-ethylamino-6-isopropylamino-1,3,5-triazin (ATRAZIN); 2-Chlor-4-ethylamino-6-(3-cyanopropylamino)-1,3,5-triazin (CYANAZIN); 2-Chlor-4,6-bis-(ethylamino)-1,3,5-triazin (SIMAZIN); 2,4-Bis-[N-ethylamino]-6-methylthio-1,3,5-triazin (SIMETRYNE); 4-Ethylamino-2-t-butylamino-6-methylthio-s-triazin (TERBUTRYNE); 4-Amino-6-t-butyl-3-ethylthio-1,2,4-triazin-5(4H)-on (ETHIOZIN); 3-Cyclohexyl-6-dimethylamino-1-methyl-1,3,5-triazin-2,4-dion (HEXAZINONE); 3-Isopropyl-2,1,3-benzothiadiazin-4-on-2,2-dioxid (BENTAZON); exo-1-Methyl-4-(1-methylethyl)-2-(2-methylphenyl-methoxy)-7-oxabicyclo(2,2,1)-heptan (CINMETHYLIN); 0-(6-Chlor-3-phenyl-pyridazin-4-yl)-S-octyl-thiocarbonat (PYRIDATE) oder 2-[(2-Chlorphenyl)-methyl]-4,4-dimethylisoxazolidin-3-on (DIMETHAZONE) sind gegebenenfalls von Vorteil. Einige Mischungen zeigen überraschenderweise auch synergistische Wirkungen.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Graninlate angewandt werden. Die Anwendung geschieht in üblicher Weise, z. B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,001 und 10 kg Wirkstoff pro Hektar Bodenfläche, vorzugsweise zwischen 0,005 und 5 kg pro Hektar.

Die Herstellung und die Verwendung der erfindungsgemäßen Wirkstoffe geht aus den nachfolgenden Beispielen hervor.

Herstellungsbeispiele:

Beispiel I-1

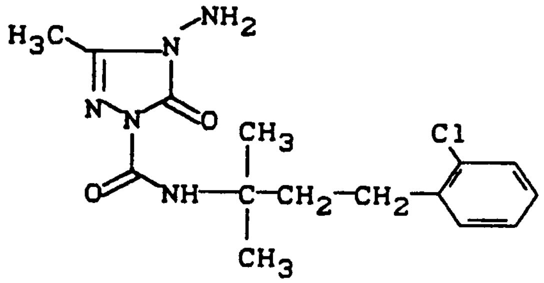

(Verfahren a)

Zu 4,2 g (0,01 Mol) 4-(4-Methylpent-2-yliden-imino)-3-methyl-1-[2-methyl-4-(2-chlorphenyl)-but-2-yl-aminocarbonyl]-1,2,4-triazolin-5-on in 100 ml Ethanol gibt man 20 ml Wasser und 5 ml konzentrierte Salzsäure und rührt 3 Stunden bei 60°C und 200 mbar. Zur Aufarbeitung wird die Lösung im Vakuum eingeengt, der Rückstand in Dichlormethan aufgenommen, mit gesättigter Natriumhydrogencarbonatlösung gewaschen, über Natriumsulfat getrocknet und im abermals im Vakuum eingeengt. Der Rückstand wir durch Verreiben mit Petrolether zur Kristallisation gebracht, abfiltriert und getrocknet.

Man erhält 1,7 g (50 % der Theorie) an 4-Amino-3-methyl-1-[2-methyl-4-(2-chlorphenyl)-but-2-yl-aminocarbonyl]-1,2,4-triazolin-5-on vom Schmelzpunkt 102°C.

Herstellung der Ausgangsverbindung:

Beispiel II-1

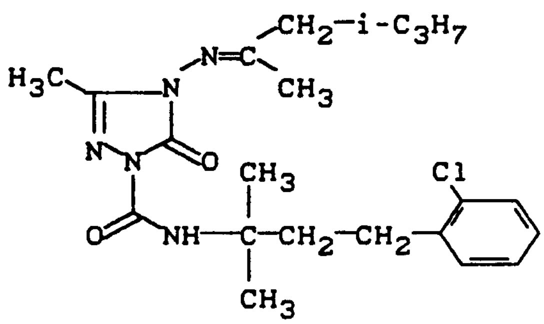

Zu 3,9 g (0,02 Mol) 4-(4-Methylpent-2-yliden-imino)-3-methyl-1,2,4-triazolin-5-on in 100 ml Acetonitril gibt man 0,2 g Diazabicycloundecen (DBU) und 4,5 g (0,02 Mol) 2-Methyl-4-(2-chlorphenyl)-but-2-yl-isocyanat und rührt 18 Stunden bei 20°C. Zur Aufarbeitung wird das Lösungsmittel im Vakuum abdestilliert, der ölige Rückstand mit Petrolether digeriert, überstehender Petrolether abdekantiert und der Rückstand im Hochvakuum von flüchtigen Anteilen befreit.

Man erhält 8,1 g (97 % der Theorie) an 4-(4-Methylpent-2-yliden-imino)-3-methyl-1-[2-methyl-4-(2-chlorphenyl)-but-2-yl-aminocarbonyl]-1,2,4-triazolin-5-on vom Schmelzpunkt 79°C.

Beispiel VIII-1

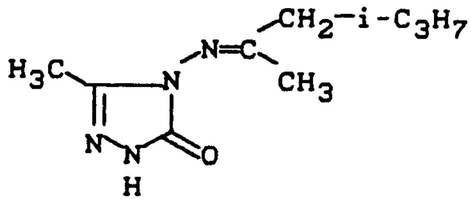

Eine Mischung von 114 g (1,0 Mol) 4-Amino-3-methyl-1,2,4-triazolin-5-on(vergleiche z. B. DE-OS 38 03 523), 2 g p-Toluolsulfonsäure und 500 ml 4-Methylpentan-2-on wird 4 Stunden über einem Wasserabscheider unter Rückfluß gekocht. Zur Aufarbeitung wird im Vakuum eingeengt, der Rückstand durch Verreiben mit Petrolether zur Kristallisation gebracht; die Kristalle werden abfiltriert und getrocknet.

Man erhält 187 g (95 % der Theorie) an 4-(4-Methylpent-2-yliden-imino)-3-methyl-1,2,4-triazolin-5-on vom Schmelzpunkt 91 ° C.

Beispiel I-2

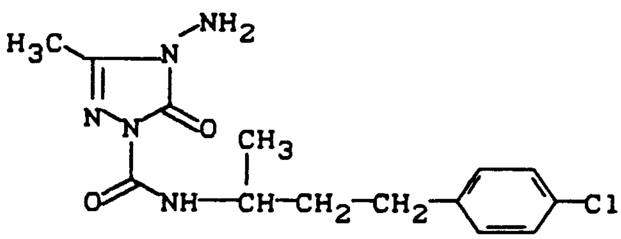

(Verfahren a)

Eine Mischung aus 6,1 g (0,015 Mol) 4-(4-Methylpent-2-yliden-imino)-3-methyl-1-[4-(4-chlorphenyl)-but-2-yl-aminocarbonyl]-1,2,4-triazolin-5-on, 50 ml Ethanol, 25 ml Wasser und 5 ml konzentrierter Salzsäure wird 3 Stunden bei 60 ° C und 200 mbar gerührt. Zur Aufarbeitung wird im Wasserstrahlvakuum eingeengt, der Rückstand in 200 ml Dichlormethan aufgenommen, mit 200 ml gesättigter wässriger Natriumhydrogencarbonatlösung gewaschen, die organische Phase über Magnesiumsulfat getrocknet, filtriert und im Vakuum vom Lösungsmittel befreit.

Man erhält 3,4 g (70 % der Theorie) an 4-Amino-3-methyl-1-[4-(4-chlorphenyl)-but-2-yl-aminocarbonyl]-1,2,4-triazolin-5-on als Öl.

$^1$H-NMR ($CDCl_3$/Tetramethylsilan): $\delta$ = 1,26 (d,J = 7 Hz); 2,33 (s) ppm.

Synthese der Ausgangsverbindung:

Beispiel II-2

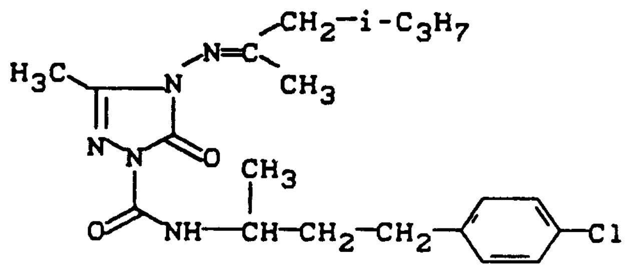

6,3 g (0,02 Mol) 4-(4-Methylpent-2-yliden-imino)-3-methyl-1-phenoxycarbonyl-1,2,4-triazolin-5-on und 3,7 g (0,02 Mol) 4-(4-Chlorphenyl)-but-2-ylamin in 100 ml Tetrahydrofuran werden 18 Stunden bei 20 °C gerührt, anschließend im Vakuum eingeengt, der Rückstand in Dichlormethan aufgenommen, mehrmals mit wässriger Natriumhydrogencarbonatlösung gewaschen, über Natriumsulfat getrocknet, filtriert und im Vakuum vom Lösungsmittel befreit.

Man erhält 7,0 g (86 % der Theorie) an 4-(4-Methylpent-2-yliden-imino)-3-methyl-1-[4-(4-chlorphenyl)-but-2-yl-aminocarbonyl]-1,2,4-triazolin-5-on als Öl.

$^1$H-NMR ($CDCl_3$/Tetramethylsilan): $\delta$ = 1,26 (d,J = 7 Hz); 2,0 (s); 2,24 (s) ppm.

Beispiel X-1

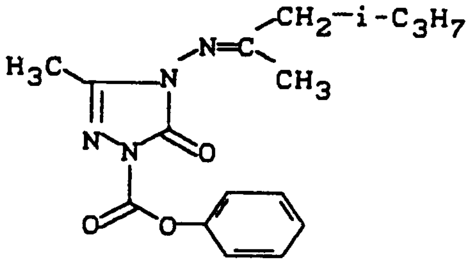

Zu einer Mischung aus 98 g (0,5 Mol) 4-(4-Methylpent-2-yliden-imino)-3-methyl-1,2,4-triazolin-5-on, 625 ml Dichlormethan, 22 g (0,55 Mol) Natriumhydroxid, 625 ml Wasser und 4 g Tetrabutylammoniumbromid gibt man tropfenweise unter Rühren 86,1 g (0,55 Mol) Chlorameisensäurephenylester, rührt anschließend 18 Stunden bei 20 °C, trennt dann die organische Phase ab, wäscht mit Wasser, trocknet über Natriumsulfat, engt im Vakuum ein. Der Rückstand wird durch verreiben mit Diethylether zur Kristallisation gebracht, die entstandenen Kristalle werden abfiltriert und getrocknet.

Man erhält 83,7 g (53 % der Theorie) an 4-(4-Methylpent-2-yliden-imino)-3-methyl-1-phenoxycarbonyl-1,2,4-triazolin-5-on vom Schmelzpunkt 114 °C.

Beispiel I-3

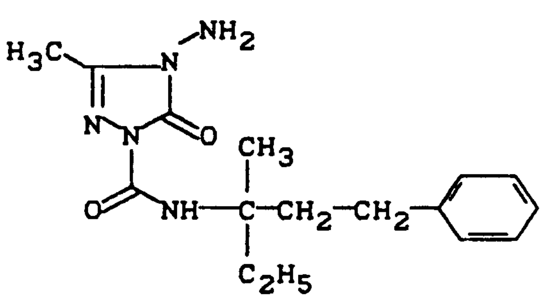

[Verfahren (a) "Eintopfvariante"]

Zu 3,9 g (0,02 Mol) 4-(4-Methylpent-2-yliden-imino)-3-methyl-1,2,4-triazolin-5-on in 100 ml Dichlormethan gibt man nacheinander 0,1 g Diazabicycloundecen (DBU) und 4,1 g (0,02 Mol) 3-Methyl-1-phenyl-pent-3-yl-isocyanat und rührt 18 Stunden bei 20° C. Anschließend wird das Lösungsmittel im Vakuum entfernt, der Rückstand in 100 ml Ethanol aufgenommen, mit 20 ml Wasser und 5 ml konzentrierter Salzsäure versetzt und 3 Stunden bei 60° C und 200 mbar gerührt. Zur Aufarbeitung wird das Lösungsmittel im Vakuum abdestilliert, der Rückstand in Dichlormethan aufgenommen, mit gesättigter wässriger Natriumhydrogencarbonatlösung gewaschen, über Natriumsulfat getrocknet und im Vakuum eingeengt. Der Rückstand wird durch Verreiben mit Petrolether zur Kristallisation gebracht, abfiltriert und getrocknet.

Man erhält 5,2 g (82 % der Theorie) an 4-Amino-3-methyl-1-(3-methyl-1-phenyl-pent-3-yl-aminocarbonyl)-1,2,4-triazolin-5-on vom Schmelzpunkt 78° C.

Beispiel I-4

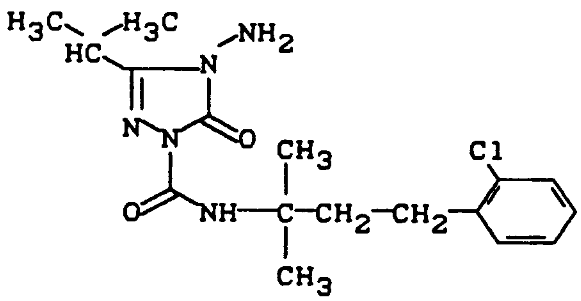

(Verfahren b)

Zu 1,4 g (0,01 Mol) 4-Amino-3-isopropyl-1,2,4-triazolin-5-on (vergleiche z. B. DE-OS 38 03 523) in 100 ml Acetonitril gibt man 1 ml Diazabicycloundecen (DBU) und 2,2 g (0,01 Mol) 2-Methyl-4-(2-chlorphenyl)-but-2-yl-isocyanat, rührt anschließend 18 Stunden bei 40° C, kühlt dann auf Raumtemperatur ab und filtriert das kristallin ausgefallene Produkt ab.

Man erhält 1,4 g (38 % der Theorie) an 4-Amino-3-isopropyl-1-[2-methyl-4-(2-chlorphenyl)-but-2-yl-aminocarbonyl]-1,2,4-triazolin-5-on vom Schmelzpunkt 159° C.

Synthese der Ausgangsverbindung:

Beispiel IV-1

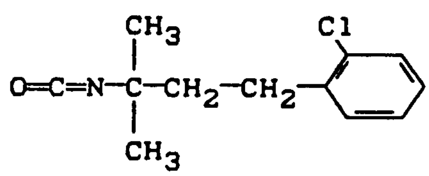

Zu 150 g (1,5 Mol) Phosgen in 2,5 l Chlorbenzol gibt man bei 15° C tropfenweise unter Rühren eine Lösung von 198 g (1,0 Mol) 1,1-Dimethyl-3-(2-chlorphenyl)-propylamin in 1,0 l Chlorbenzol, wobei die Temperatur der Reaktionsmischung auf 25° C ansteigt. Nach beendeter Zugabe wird auf 70° C erwärmt und unter Rühren und weiterem Einleiten von Phosgen (ca. 50 g/Stunde) langsam auf Rückflußtemperatur erhitzt und noch weitere 30 Minuten bei dieser Temperatur gerührt. Zur Aufarbeitung wird ca. 1 l Chlorbenzol zusammen mit überschüssigem Phosgen abdestilliert, der Rückstand im Wasserstrahlvakuum eingeengt und im Hochvakuum destilliert.

Man erhält 212 g (95 % der Theorie) an 2-Methyl-4-(2-chlorphenyl)-but-2-yl-isocyanat vom Siedepunkt

83 ° C bei 0,1 mbar.

Beispiel VI-1

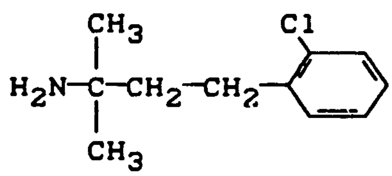

40,6 g (0,21 Mol) 3-Amino-3,3-dimethyl-1-(2-chlorphenyl)-1-propin werden zusammen mit 10 g Raney-Nickel und 250 ml Tetrahydrofuran in einem Rührautoklaven mit Wasserstoff bis zu einem Druck von 50 bar versetzt und dann allmählich unter Rühren auf 40 ° C erwärmt, wobei der Wasserstoffdruck auf 50 bar konstant gehalten wird. Zur Aufarbeitung wird filtriert, das Filtrat im Wasserstrahlvakuum eingeengt und der Rückstand im Hochvakuum destilliert.

Man erhält 31,8 g (77 % der Theorie) an 3-Amino-3,3-dimethyl-1-(2-chlorphenyl)-1-propan vom Brechungsindex $n_D^{21}$ = 1,4817.

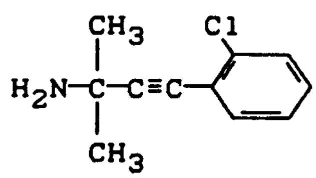

Zu 96,7 g (0,5 Mol) 1-Brom-2-chlor-benzol und 45,7 g (0,55 Mol) 3-Amino-3,3-dimethyl-1-propin in 500 ml Triethylamin gibt man 7,0 g (0,01 Mol) Palladium-(II)-bis-triphenylphosphin-dichlorid, 7,6 g (0,04 Mol) Kupfer-(I)-iodid und 21,0 g (0,4 Mol) Triphenylphosphin, erhitzt für 24 Stunden auf Rückflußtemperatur, filtriert anschließend und engt das Filtrat im Wasserstrahlvakuum ein. Der Rückstand wird zwischen Dichlormethan (ca. 300 ml) und Wasser (ca. 300 ml) verteilt, die organische Phase abgetrennt, über Natriumsulfat getrocknet und filtriert. Das Filtrat wird im Wasserstrahlvakuum eingeengt und im Hochvakuum destilliert.

Man erhält 74,1 g (75 % der Theorie) an 3-Amino-3,3-dimethyl-1-(2-chlorphenyl)-1-propin vom Brechungsindex $n_D^{21}$ = 1,5799.

In entsprechender Weise und gemäß den allgemeinen Angaben zur Synthese erhält man die folgenden substituierten Triazolinone der allgemeinen Formel (I):

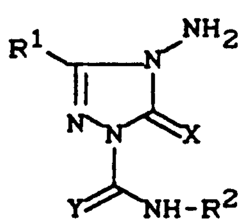

(I)

| Bsp. Nr. | $R^1$ | $R^2$ | X | Y | physikalische Eigenschaften |
|---|---|---|---|---|---|
| I-5 | $C_2H_5$ | 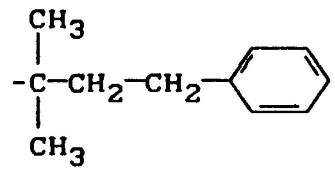 | O | O | $^1$H-NMR*): 1,46; 2,03-2,13; 2,6-2,7 |
| I-6 | 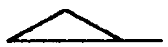 | 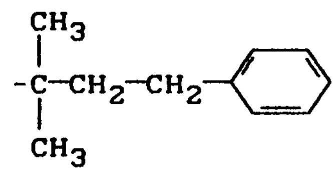 | O | O | Fp. 129° C |
| I-7 | $i$-$C_3H_7$ | 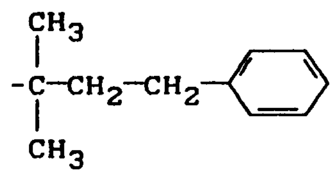 | O | O | Fp. 87° C |
| I-8 | $i$-$C_3H_7$ | 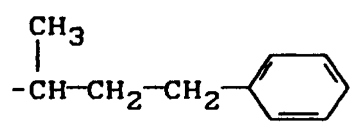 | O | O | $n_D^{20}$ = 1,5365 |
| I-9 | $i$-$C_3H_7$ | 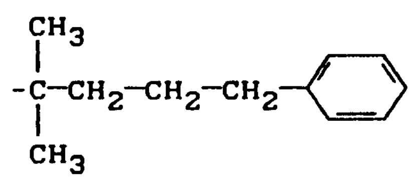 | O | O | Fp. 79° C |

| Bsp. Nr. | $R^1$ | $R^2$ | X | Y | physikalische Eigenschaften |
|---|---|---|---|---|---|
| I-10 | $CH_3$ | 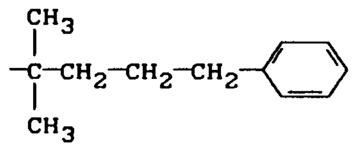 | O | O | $^1$H-NMR*): 1,38; 2,3 |
| I-11 | $CH_3$ | 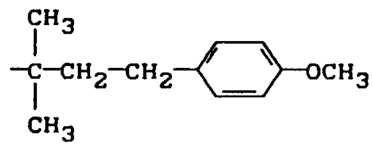 | O | O | $^1$H-NMR*): 1,44; 3,27; 3,75 |
| I-12 | i-$C_3H_7$ | 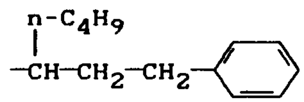 | O | O | $n_D^{20}$ = 1,5209 |
| I-13 | $CH_3$ | 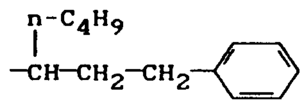 | O | O | Fp. 84° C |
| I-14 | $CH_3$ | 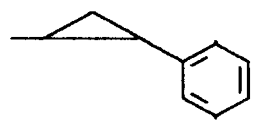 | O | O | Fp. 152° C |
| I-15 | $CH_3$ | 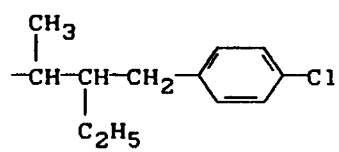 | O | O | Fp. 122-123° C |

| Bsp. Nr. | $R^1$ | $R^2$ | X | Y | physikalische Eigenschaften |
| --- | --- | --- | --- | --- | --- |
| I-16 | $CH_3$ | 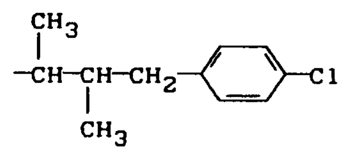 | O | O | Fp. 118-119° C |
| I-17 | $CH_3$ | 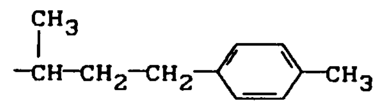 | O | O | $^1$H-NMR*): 1,25; 2,30; 2,31 |
| I-18 | $CH_3$ | 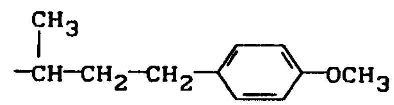 | O | O | $^1$H-NMR*): 2,33; 3,77 |
| I-19 | $CH_3$ | 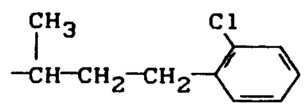 | O | O | $^1$H-NMR*): 1,28; 2,33 |
| I-20 | $CH_3$ | 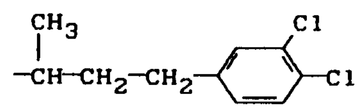 | O | O | $^1$H-NMR*): 1,26; 2,34 |
| I-21 | $CH_3$ | 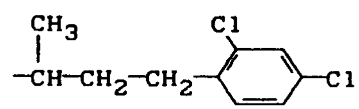 | O | O | Fp. 98° C |

| Bsp. Nr. | $R^1$ | $R^2$ | X | Y | physikalische Eigenschaften |
|---|---|---|---|---|---|
| 1-22 | $CH_3$ | 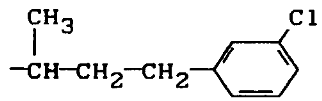 | O | O | $^1H$-NMR*): 1,27; 2,32 |
| I-23 | $CH_3$ | 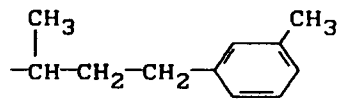 | O | O | $^1H$-NMR*): 1,26; 2,31; 2,32 |
| I-24 | $CH_3$ | 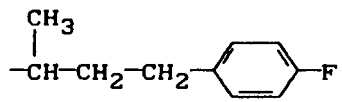 | O | O | $^1H$-NMR*): 1,26; 2,33 |
| I-25 | $CH_3$ | 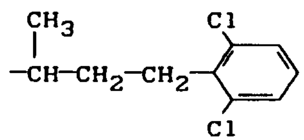 | O | O | Fp. 75° C |
| I-26 | $CH_3$ | 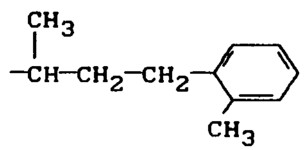 | O | O | Fp. 94° C |
| I-27 | $i-C_3H_7$ | 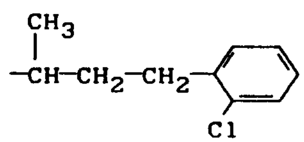 | O | O | $^1H$-NMR*): 1,28; 1,35 |

| Bsp. Nr. | $R^1$ | $R^2$ | X | Y | physikalische Eigenschaften |
|---|---|---|---|---|---|
| I-28 | $i-C_3H_7$ | 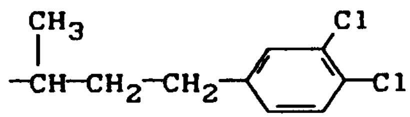 | O | O | $^1H$-NMR*): 1,26; 1,35 |
| I-29 | $i-C_3H_7$ | 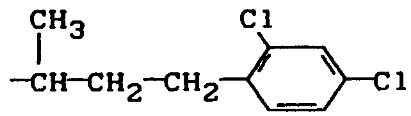 | O | O | $^1H$-NMR*): 1,27; 1,34 |
| I-30 | $i-C_3H_7$ | 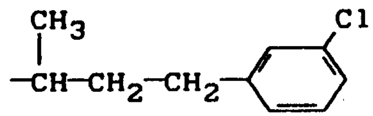 | O | O | $^1H$-NMR*): 1,27; 1,35 |
| I-31 | $i-C_3H_7$ | 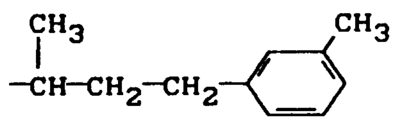 | O | O | $^1H$-NMR*): 1,27; 1,34; 2,31 |
| I-32 | $i-C_3H_7$ | 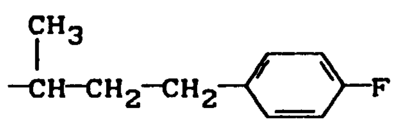 | O | O | $^1H$-NMR*): 1,26; 1,35 |
| I-33 | $i-C_3H_7$ | 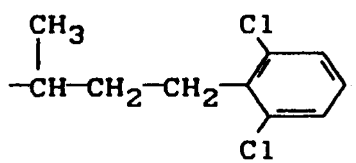 | O | O | $^1H$-NMR*): 1,34; 1,7-1,95; 2,9-3,22 |

| Bsp. Nr. | $R^1$ | $R^2$ | X | Y | physikalische Eigenschaften |
|---|---|---|---|---|---|
| I-34 | $i-C_3H_7$ | 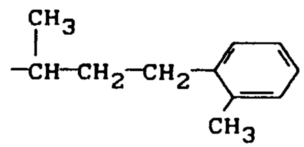 | O | O | $^1$ H-NMR*): 1,29; 1,34; 2,30 |
| I-35 | 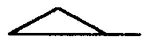 | 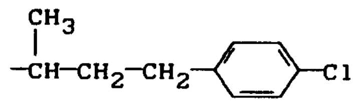 | O | O | Fp. 110° C |
| I-36 | 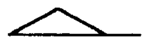 | 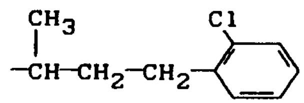 | O | O | Fp. 125° C |
| I-37 | 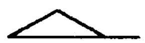 | 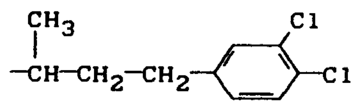 | O | O | $^1$ H-NMR*): 1,24 |
| I-38 | 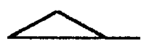 | 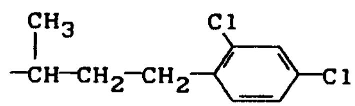 | O | O | Fp. 81° C |
| I-39 | 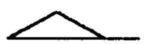 | 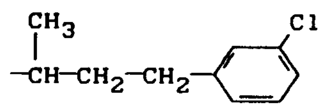 | O | O | $^1$ H-NMR*): 1,24 |

| Bsp. Nr. | $R^1$ | $R^2$ | X | Y | physikalische Eigenschaften |
|---|---|---|---|---|---|
| I-40 | 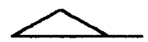 | 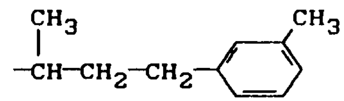 | O | O | $^1$H-NMR*): 1,24 |
| I-41 | 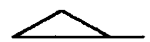 | 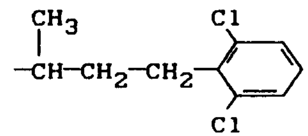 | O | O | Fp. 118° C |
| I-42 | 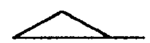 | 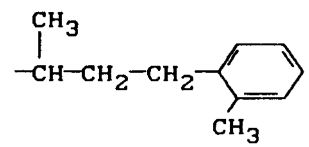 | O | O | Fp. 117° C |
| I-43 | 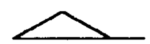 | 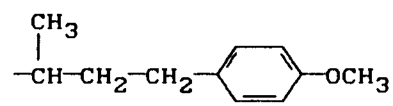 | O | O | $^1$H-NMR*): 3,71 |
| I-44 | i-$C_3H_7$ | 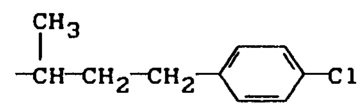 | O | O | $^1$H-NMR*): 1,27; 1,35 |
| I-45 | 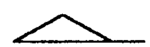 | 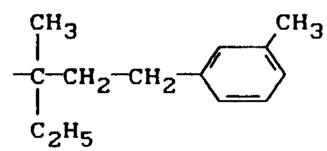 | O | O | $^1$H-NMR*): 1,40; 2,30 |

| Bsp. Nr. | $R^1$ | $R^2$ | X | Y | physikalische Eigenschaften |
|---|---|---|---|---|---|
| I-46 | $i-C_3H_7$ | 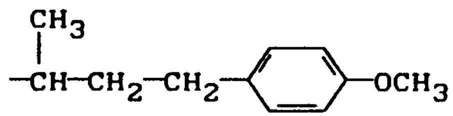 | O | O | $^1$H-NMR*): 3,66 |
| I-47 | $i-C_3H_7$ | 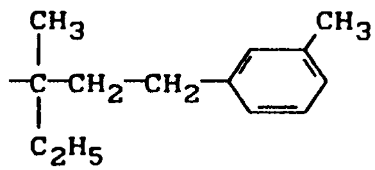 | O | O | $^1$H-NMR*): 1,40; 2,31 |
| I-48 | $CH_3$ | 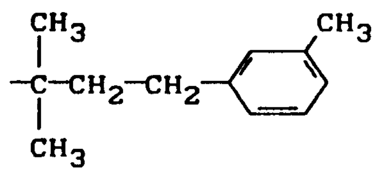 | O | O | $^1$H-NMR*): 1,45; 2,29; 2,30 |
| I-49 | $i-C_3H_7$ | 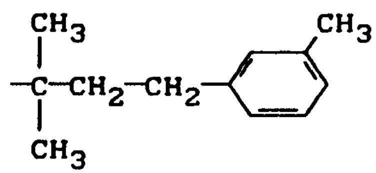 | O | O | $^1$H-NMR*): 1,45; 2,31 |
| I-50 | 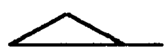 | 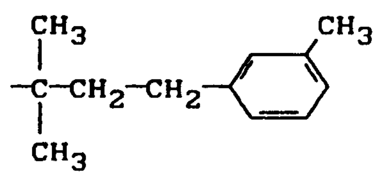 | O | O | Fp. 107° C |

| Bsp. Nr. | $R^1$ | $R^2$ | X | Y | physikalische Eigenschaften |
|---|---|---|---|---|---|
| I-51 | $C_2H_5$ | 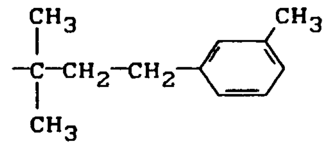 | O | O | $^1$H-NMR*): 1,45; 2,31 |
| I-52 | $C_2H_5$ | 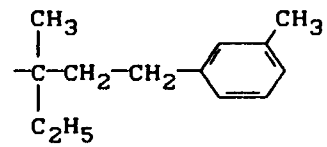 | O | O | $^1$H-NMR*): 1,41; 2,31 |
| I-53 | i-$C_3H_7$ | 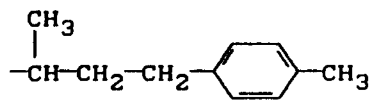 | O | O | $^1$H-NMR*): 1,26; 1,35 |
| I-54 | 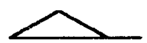 | 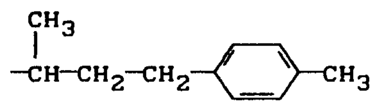 | O | O | $^1$H-NMR*): 1,24; 2,29 |
| I-55 | 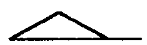 | 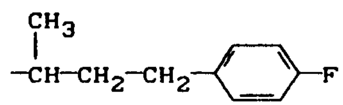 | O | O | Fp. 107° C |
| I-56 | $CH_3$ | 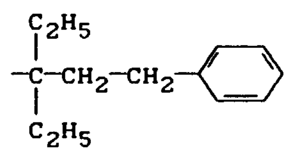 | O | O | Fp. 89° C |

| Bsp. Nr. | $R^1$ | $R^2$ | X | Y | physikalische Eigenschaften |
|---|---|---|---|---|---|
| I-57 | $CH_3$ | 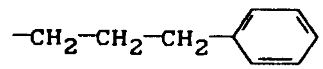 | O | O | Fp. 65° C |
| I-58 | $CH_3$ | 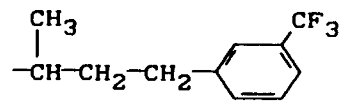 | O | O | $^1$H-NMR*): 2,25 |
| I-59 | i-$C_3H_7$ | 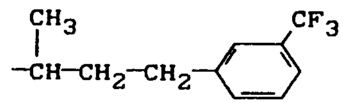 | O | O | $^1$H-NMR*): 1,36; 1,8-1,95 2,7-2,8 |
| I-60 | i-$C_3H_7$ | 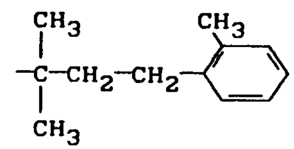 | O | O | Fp. 86° C |
| I-61 | i-$C_3H_7$ | 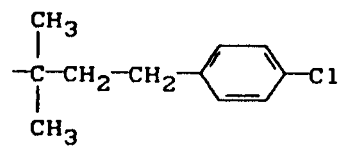 | O | O | Fp. 130° C |
| I-62 | i-$C_3H_7$ | 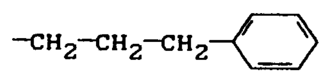 | O | O | Fp. 79° C |

| Bsp. Nr. | $R^1$ | $R^2$ | X | Y | physikalische Eigenschaften |
|---|---|---|---|---|---|
| I-63 | $CH_3$ | 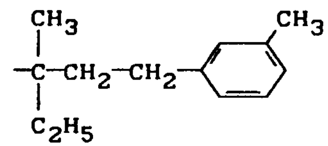 | O | O | $^1$H-NMR*): 1,39; 2,28; 2,30 |
| I-64 | $CH_3$ | 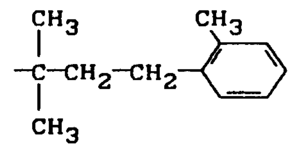 | O | O | Fp. 122°C |
| I-65 | $CH_3$ | 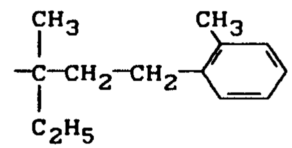 | O | O | Fp. 118°C |
| I-66 | $CH_3$ | 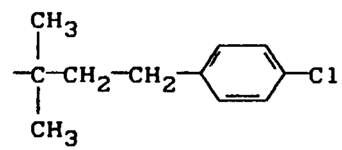 | O | O | Fp. 125°C |
| I-67 | $CH_3$ | 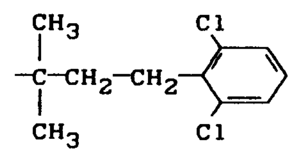 | O | O | Fp. 156°C |

| Bsp. Nr. | $R^1$ | $R^2$ | X | Y | physikalische Eigenschaften |
|---|---|---|---|---|---|
| I-68 | $CH_3$ | 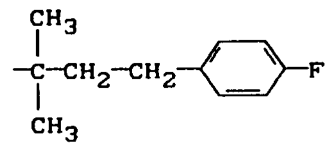 | O | O | Fp. 161° C |
| I-69 | $CH_3$ | 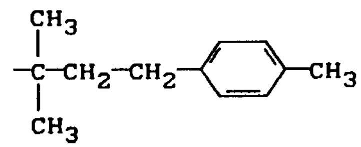 | O | O | Fp. 109° C |
| I-70 | $CH_3$ | 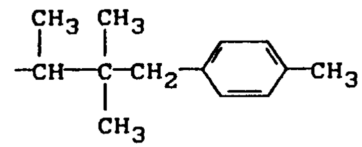 | O | O | Fp. 80° C |
| I-71 | $CH_3$ | 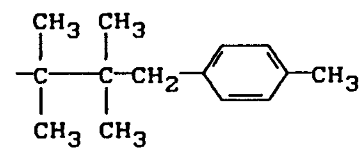 | O | O | Fp. 137° C |
| I-72 | $CH_3$ | 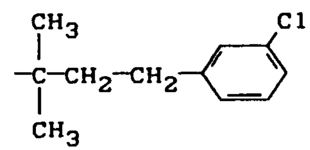 | O | O | Fp. 110° C |

| Bsp. Nr. | $R^1$ | $R^2$ | X | Y | physikalische Eigenschaften |
|---|---|---|---|---|---|
| I-73 | $CH_3$ | 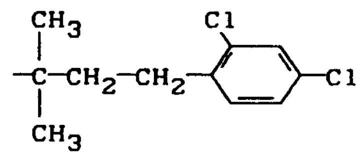 | O | O | Fp. 143° C |
| I-74 | $CH_3$ | 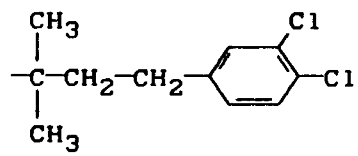 | O | O | Fp. 119° C |
| I-75 | i-$C_3H_7$ | 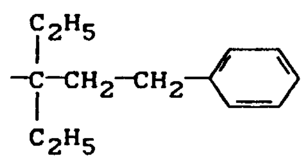 | O | O | Fp. 94° C |
| I-76 | i-$C_3H_7$ | 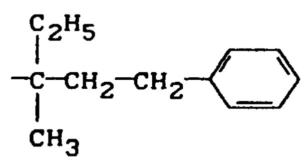 | O | O | Fp. 87° C |
| I-77 | i-$C_3H_7$ | 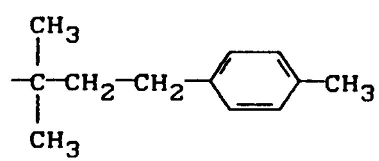 | O | O | Fp. 127° C |

| Bsp. Nr. | $R^1$ | $R^2$ | X | Y | physikalische Eigenschaften |
|---|---|---|---|---|---|
| I-78 | i-$C_3H_7$ | 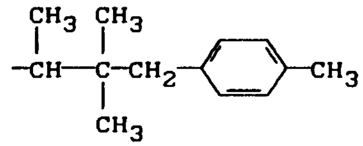 | O | O | Fp. 121° C |
| I-79 | i-$C_3H_7$ | 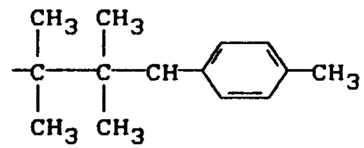 | O | O | Fp. 86° C |
| I-80 | i-$C_3H_7$ | 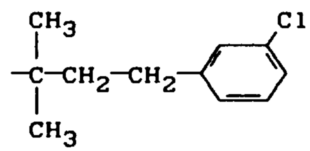 | O | O | Fp. 78° C |
| I-81 | i-$C_3H_7$ | 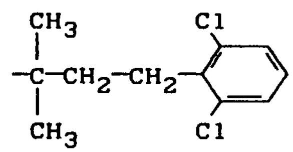 | O | O | Fp. 124° C |
| I-82 | i-$C_3H_7$ | 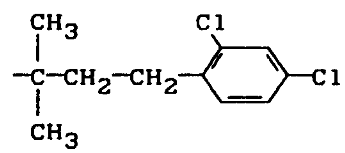 | O | O | Fp. 123° C |

| Bsp. Nr. | $R^1$ | $R^2$ | X | Y | physikalische Eigenschaften |
|---|---|---|---|---|---|
| I-83 | i-$C_3H_7$ | 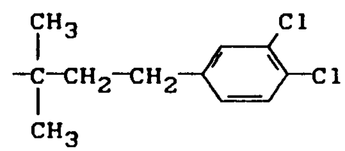 | O | O | Fp. 78° C |
| I-84 | i-$C_3H_7$ | 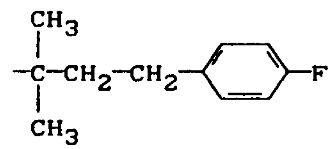 | O | O | Fp. 95° C |
| I-85 | 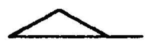 | 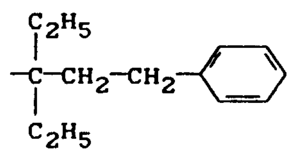 | O | O | $^1$H-NMR*): 2,4-2,6 |
| I-86 | 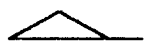 | 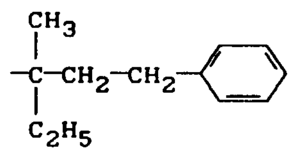 | O | O | $^1$H-NMR*): 1,39 |
| I-87 | 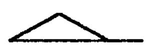 | 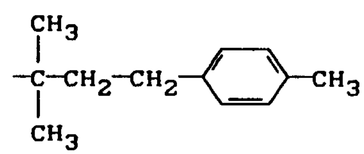 | O | O | Fp. 144° C |

| Bsp. Nr. | $R^1$ | $R^2$ | X | Y | physikalische Eigenschaften |
|---|---|---|---|---|---|
| I-88 | $CH_3$ | 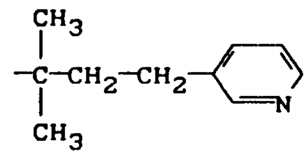 | O | O | $^1H$-NMR*): 1,48; 2,31 |
| I-89 | i-$C_3H_7$ | 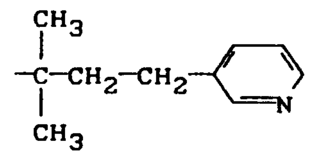 | O | O | $^1H$-NMR*): 1,34; 1,45 |
| I-90 | 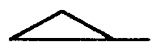 | 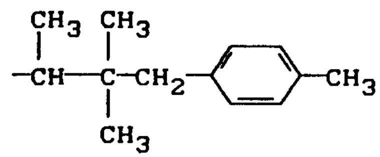 | O | O | Fp. 76° C |
| I-91 | 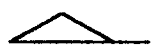 | 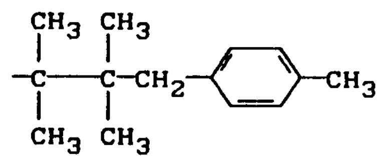 | O | O | Fp. 76° C |
| I-92 | 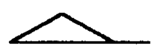 | 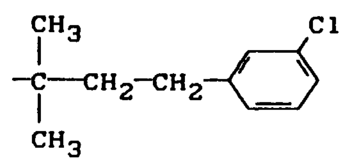 | O | O | Fp. 95° C |

| Bsp. Nr. | $R^1$ | $R^2$ | X | Y | physikalische Eigenschaften |
|---|---|---|---|---|---|
| I-93 | 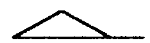 | 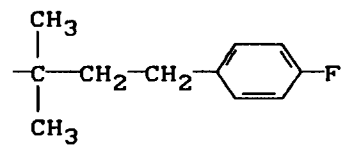 | O | O | Fp. 135° C |
| I-94 | $C_2H_5$ | 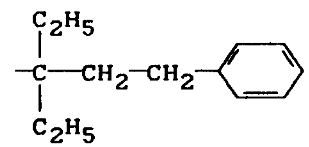 | O | O | $^1$H-NMR*): 1,31; 2,80 |
| I-95 | $C_2H_5$ | 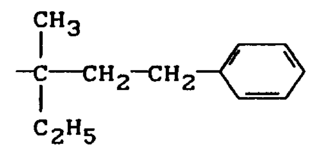 | O | O | $^1$H-NMR*): 140 |
| I-96 | $C_2H_5$ | 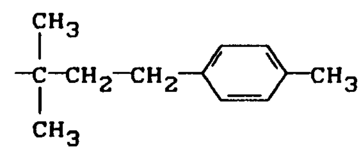 | O | O | Fp. 103° C |
| I-97 | $C_2H_5$ | 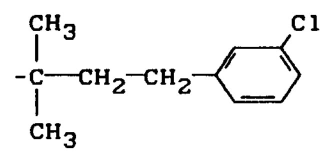 | O | O | Fp. 88° C |

| Bsp. Nr. | $R^1$ | $R^2$ | X | Y | physikalische Eigenschaften |
|---|---|---|---|---|---|
| I-98 | $C_2H_5$ | 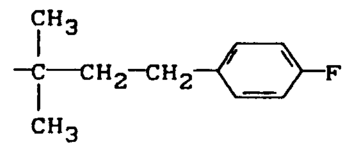 | O | O | Fp. 116°C |
| I-99 | $C_2H_5$ | 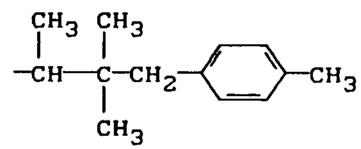 | O | O | Fp. 114°C |
| I-100 | $C_2H_5$ | 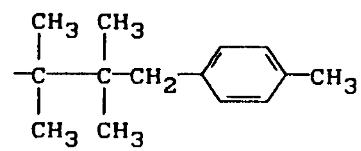 | O | O | $^1$H-NMR*): 2,32 |
| I-101 | $C_2H_5$ | 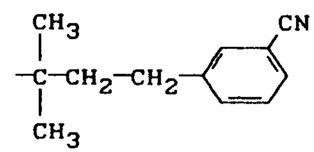 | O | O | Fp. 152°C |
| I-102 | $CH_3$ | 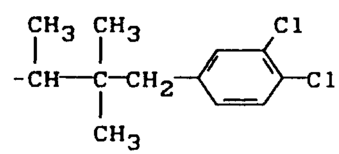 | O | O | $^1$H-NMR*): 3,05-3,2; 3,9-4,05 |

| Bsp. Nr. | $R^1$ | $R^2$ | X | Y | physikalische Eigenschaften |
|---|---|---|---|---|---|
| I-103 | $CH_3$ | 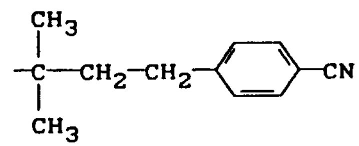 | O | O | Fp. 149-150° C |
| I-104 | $CH_3$ | 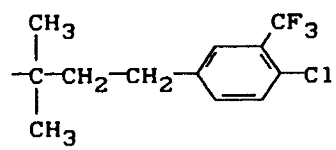 | O | O | Fp. 152° C |
| I-105 | $CH_3$ | 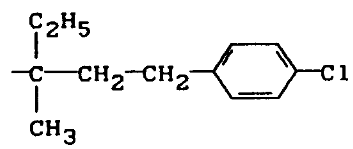 | O | O | Fp. 80° C |
| I-106 | $CH_3$ | 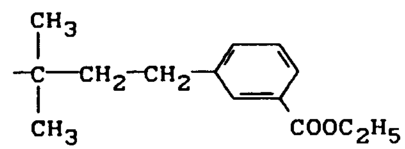 | O | O | $^1$H-NMR*): 1,46; 2,29 |
| I-107 | $CH_3$ | 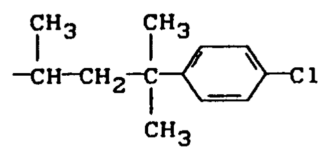 | O | O | $^1$H-NMR*): 1,27; 1,43; 2,3 |

| Bsp. Nr. | $R^1$ | $R^2$ | X | Y | physikalische Eigenschaften |
|---|---|---|---|---|---|
| I-108 | $CH_3$ | 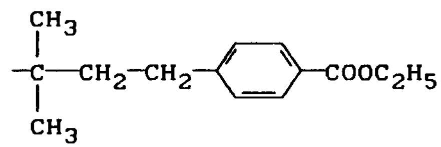 | O | O | $^1$H-NMR*): 1,45; 2,28 |
| I-109 | $CH_3$ | 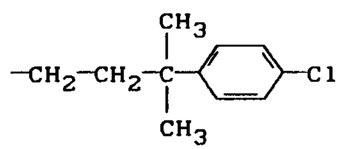 | O | O | Fp. 58°C (Zers.) |
| I-110 | i-$C_3H_7$ | 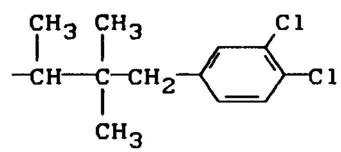 | O | O | Fp. 60°C (Zers.) |
| I-111 | $CH_3$ | 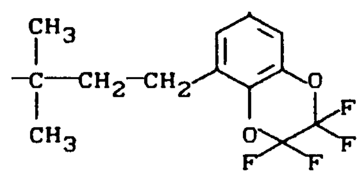 | O | O | Fp. 71-74°C |
| I-112 | $CH_3$ | 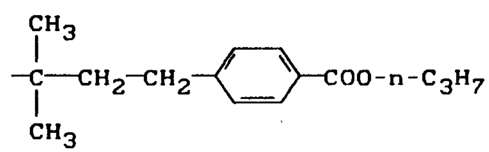 | O | O | $^1$H-NMR*): 2,27; 4,20-4,30 |

| Bsp. Nr. | $R^1$ | $R^2$ | | X | Y | physikalische Eigenschaften |
|---|---|---|---|---|---|---|
| I-113 | $CH_3$ | 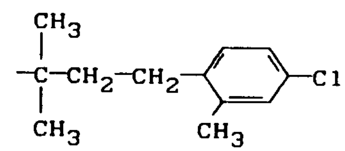 | | O | O | Fp. 131° C |
| I-114 | $CH_3$ | 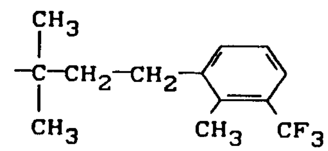 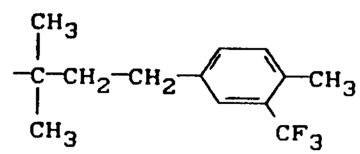 | 1:3 | O | O | Fp. 143° C |
| I-115 | $CH_3$ | 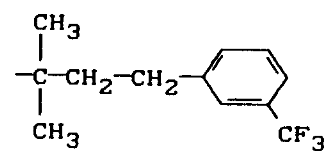 | | O | O | Fp. 127° C |
| I-116 | $CH_3$ | 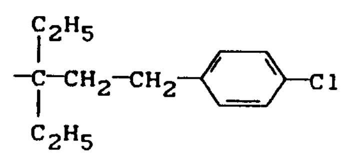 | | O | O | Fp. 112° C |

| Bsp. Nr. | $R^1$ | $R^2$ | X | Y | physikalische Eigenschaften |
|---|---|---|---|---|---|
| I-117 | $CH_3$ | 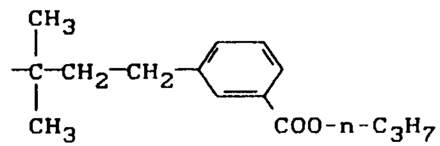 | O | O | ¹H-NMR*): 1,47; 2,31 |
| I-118 | $CH_3$ | 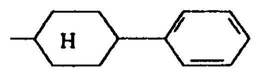 | O | O | Fp. 156° C |
| I-119 | i-$C_3H_7$ | 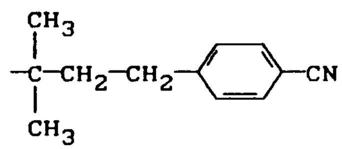 | O | O | Fp. 138-140° C |
| I-120 | $CH_3$ | 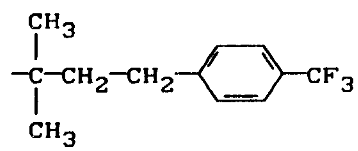 | O | O | Fp. 122° C |
| I-121 | $CH_3$ | 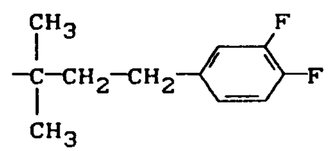 | O | O | Fp. 118° C |

| Bsp. Nr. | $R^1$ | $R^2$ | X | Y | physikalische Eigenschaften |
|---|---|---|---|---|---|
| I-122 | $i-C_3H_7$ | 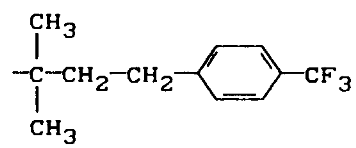 | O | O | Fp. 146° C |
| I-123 | $i-C_3H_7$ | 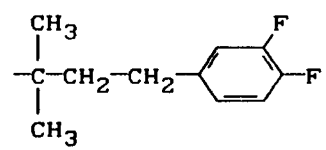 | O | O | Fp. 93° C |
| I-124 | $CH_3$ | 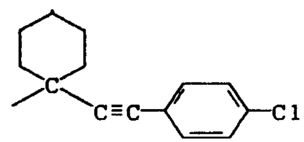 | O | O | Fp. 164° C |
| I-125 | $i-C_3H_7$ | 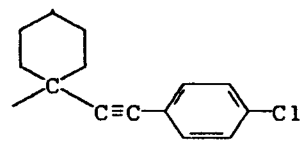 | O | O | Fp. 145° C |
| I-126 | $i-C_3H_7$ | 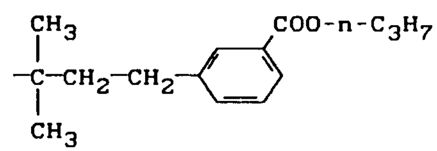 | O | O | $^1H$-NMR*): 0,95-1,05; 1,46 |

| Bsp. Nr. | $R^1$ | $R^2$ | X | Y | physikalische Eigenschaften |
|---|---|---|---|---|---|
| I-127 | i-$C_3H_7$ | 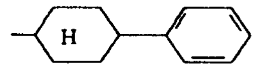 | O | O | Fp. 105° C |
| I-128 | i-$C_3H_7$ | 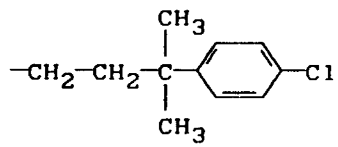 | O | O | Fp. 117° C |
| I-129 | i-$C_3H_7$ | 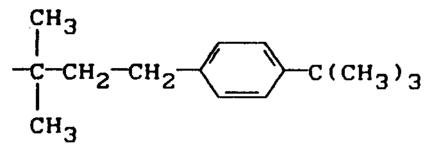 | O | O | Fp. 110° C |
| I-130 | $CH_3$ | 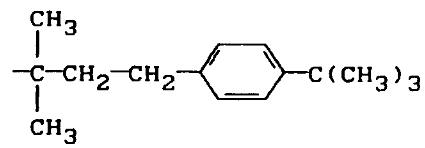 | O | O | Fp. 120° C |
| I-131 | i-$C_3H_7$ | 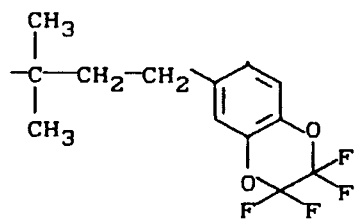 | O | O | Fp. 118° C |

| Bsp. Nr. | $R^1$ | $R^2$ | | X | Y | physikalische Eigenschaften |
|---|---|---|---|---|---|---|
| I-132 | $i-C_3H_7$ | 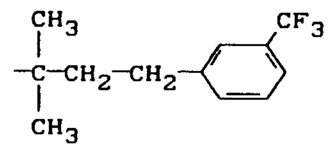 | | O | O | Fp. 81° C |
| I-133 | $i-C_3H_7$ | 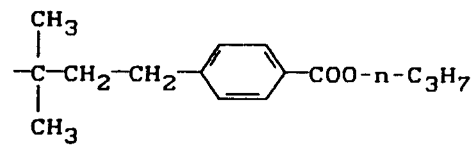 | | O | O | $^1$H-NMR*): 1,46; 1,70-1,90 |
| I-134 | $i-C_3H_7$ | 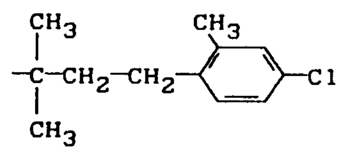 | | O | O | Fp. 129° C |
| I-135 | $i-C_3H_7$ | 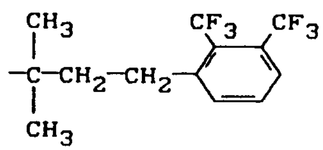 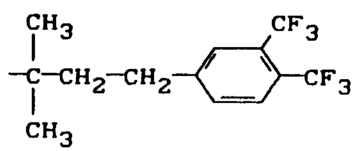 | 1:3 | O | O | Fp. 119° C |

| Bsp. Nr. | $R^1$ | $R^2$ | X | Y | physikalische Eigenschaften |
|---|---|---|---|---|---|
| I-136 | $i\text{-}C_3H_7$ | 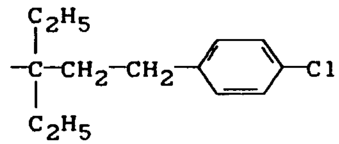 | O | O | Fp. 142° C |
| I-137 | $i\text{-}C_3H_7$ | 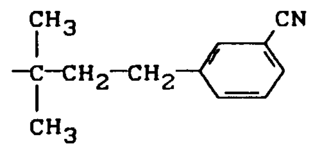 | O | O | Fp. 98-99° C |
| I-138 | $i\text{-}C_3H_7$ | 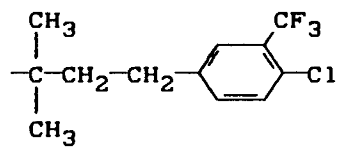 | O | O | Fp. 103° C |
| I-139 | $i\text{-}C_3H_7$ | 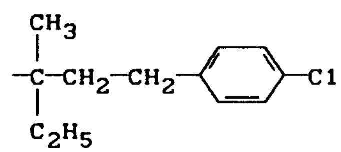 | O | O | Fp. 127° C |
| I-140 | $i\text{-}C_3H_7$ | 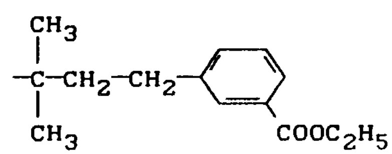 | O | O | $^1$H-NMR*): 1,46; 4,3-4,42 |

| Bsp. Nr. | $R^1$ | $R^2$ | X | Y | physikalische Eigenschaften |
|---|---|---|---|---|---|
| I-141 | $i-C_3H_7$ | 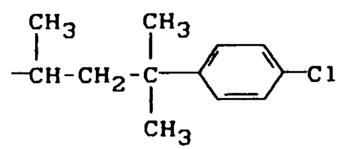 | O | O | $^1$H-NMR*): 3,04-3,2; 4,25 |
| I-142 | $i-C_3H_7$ | 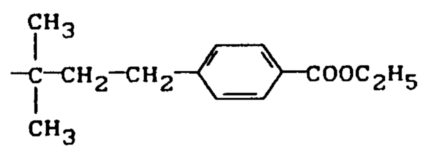 | O | O | $^1$H-NMR*): 1,44; 3,02-3,17 |
| I-143 | $i-C_3H_7$ | 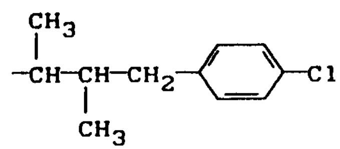 | O | O | Fp. 117° C |
| I-144 | $C_2H_5$ | 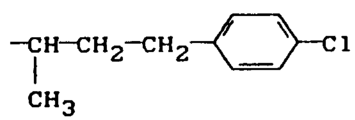 | O | O | $^1$H-NMR*): 1,82-1,9; 2,65-2,75; 7,1-7,15; 7,6-7,65 |

*) Die $^1$H-NMR-Spektren wurden in Deuterochloroform ($CDCl_3$) oder Hexadeuterodimethylsufoxid ($DMSO-d_6$) mit Tetramethylsilan (TMS) als innerem Standard aufgenommen. Angegeben ist die chemische Verschiebung als δ-Wert in ppm.

| Beispiel-Nr. | $R^1$ | $R^2$ | X | Y | physikalische Eigenschaften |
|---|---|---|---|---|---|
| 145 | $CH_3$ | 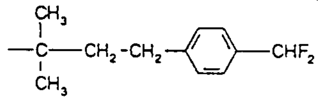 | O | O | $^1$H-NMR*): 1,45; 4,32; 6,39; 6,58; 6,77. |
| 146 | $CH_3$ | 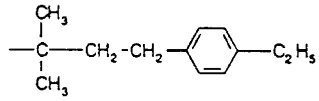 | O | O | Fp. 106°C |
| 147 | i-$C_3H_7$ | 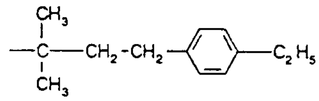 | O | O | Fp. 133°C |
| 148 | i-$C_3H_7$ | 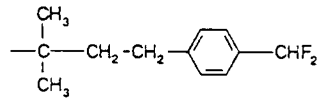 | O | O | Fp. 131°C |
| 149 | $CH_3$ | 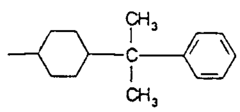 | O | O | $^1$H-NMR*): 1,27; 2,28; 4,55. |
| 150 | i-$C_3H_7$ | 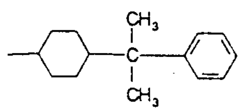 | O | O | $^1$H-NMR*): 3,0-3,2; 4,47; 7,1-7,35. |
| 151 | i-$C_3H_7$ | 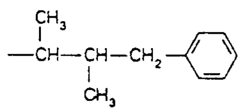 | O | O | $^1$H-NMR*): 0,85-0,93; 1,33-1,38 4,35; 7,15-7,30. |
| 152 | $CH_3$ | 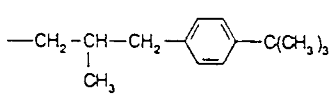 | O | O | Fp. 123-125°C |

Anwendungsbeispiele:

In den folgenden Anwendungsbeispielen wurde die nachstehend aufgeführte Verbindung als Vergleichssubstanz eingesetzt:

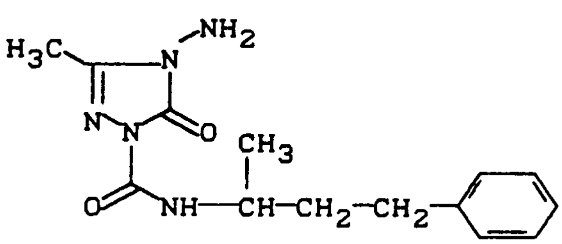

(A)

1-(Phenyl-2-butylaminocarbonyl)-3-methyl-4-amino-1,2,4-triazolin-5-on (vergleiche z. B. DE-OS 37 19 575)

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Eine deutliche Überlegenheit in der Wirksamkeit ebenso wie in der Nutzpflanzenselektivität gegenüber dem Stand der Technik zeigen in diesem Test z.B. die Verbindungen gemäß folgender Herstellungsbeispiele: I-9, I-32, I-35 und I-59.

Beispiel B

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben, so, daß die jeweils gewünschten Wirkstoffmengen pro Flächeneinheit ausgebracht werden. Die Konzentration der Spritzbrühe wird so gewählt, daß in 1000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:

0 % = keine Wirkung (wie unbehandelte Kontrolle)
100 % = totale Vernichtung

Eine deutliche Überlegenheit in der Wirksamkeit ebenso wie in der Nutzpflanzenselektivität gegenüber dem Stand der Technik zeigen in diesem Test z.B. die Verbindungen der Formel I gemäß folgender Herstellungsbeispiele: I-2, I-5, I-7, I-8, I-9, I-10, I-18, I-22, I-30, I-31, I-32, I-35, I-37, I-39, I-43, I-44, I-48, I-53, I-54, I-55, I-58, I-59, I-66, I-69, I-72, I-73, I-74, I-76, I-80, I-83 und I-84.

**Patentansprüche**

**1.** Substituierte Triazolinone der allgemeinen Formel (I)

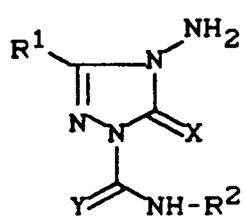

(I)

bei welchen

$R^1$ für Alkyl oder Cycloalkyl steht,

$R^2$ für gegebenenfalls durch Aryl, Arylalkyl, Arylalkenyl oder Arylalkinyl substituiertes Cycloalkyl steht, oder für einen Rest der Formel

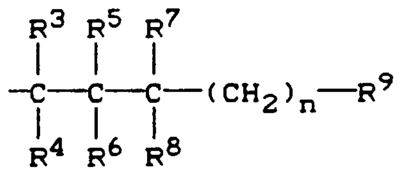

steht,

X für Sauerstoff oder Schwefel steht und

Y für Sauerstoff oder Schwefel steht,

wobei

$R^3$, $R^4$, $R^5$, $R^6$, $R^7$ und $R^8$ für Wasserstoff oder Alkyl stehen,

$R^9$ für jeweils gegebenenfalls substituiertes Cycloalkyl, Aryl oder Heteroaryl steht und

n für eine Zahl 0, 1, 2 oder 3 steht,

wobei jedoch Verbindungen, bei denen gleichzeitig $R^1$ für einen Methylrest, X für Sauerstoff, Y für Sauerstoff und $R^2$ für einen Rest der Formel

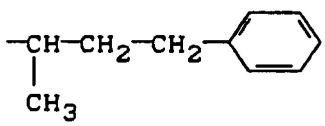 ; 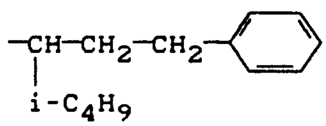 ;

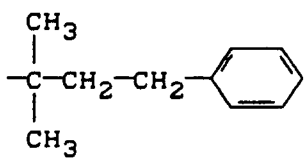 oder 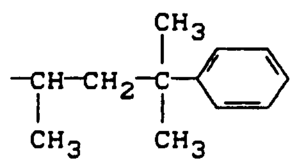

steht, ausgenommen sind.

2. Substituierte Triazolinone der allgemeinen Formel (I) gemäß Anspruch 1

bei welchen

$R^1$ für geradkettiges oder verzweigtes Alkyl mit 1 bis 6 Kohlenstoffatomen oder für Cycloalkyl mit 3 bis 7 Kohlenstoffatomen steht,

$R^2$ für gegebenenfalls einfach oder mehrfach, gleich oder verschieden substituiertes Cycloalkyl mit 3 bis 7 Kohlenstoffatomen steht, wobei als Substituenten infrage kommen:
im Arylteil jeweils gegebenenfalls einfach oder mehrfach, gleich oder verschieden substituiertes Aryl, Arylalkyl, Arylalkenyl oder Arylalkinyl mit jeweils 6 oder 10 Kohlenstoffatomen im Arylteil und gegebenenfalls bis zu 6 Kohlenstoffatomen in den jeweiligen geradkettigen oder verzweigten Alkyl- bzw. Alkenyl-oder Alkinylteilen, wobei als Arylsubstituenten jeweils infrage kommen:
Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen sowie gegebenenfalls einfach oder mehrfach, gleich oder verschieden durch Halogen und/oder geradkettiges oder verzweigtes Alkyl mit 1 bis 4 Kohlenstoffatomen substituiertes Phenyl,

$R^2$ außerdem für einen Rest der Formel

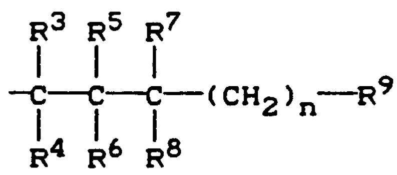

steht,

X für Sauerstoff oder Schwefel steht und

Y für Sauerstoff oder Schwefel steht, wobei

$R^3$ für geradkettiges oder verzweigtes Alkyl mit 1 bis 8 Kohlenstoffatomen steht,

$R^4$, $R^5$, $R^6$, $R^7$ und $R^8$ unabhängig voneinander jeweils für Wasserstoff oder für geradkettiges oder verzweigtes Alkyl mit 1 bis 4 Kohlenstoffatomen stehen,

$R^9$ für gegebenenfalls einfach oder mehrfach, gleich oder verschieden substituiertes Cycloalkyl mit 3 bis 7 Kohlenstoffatomen steht, wobei als Substituenten infrage kommen:
Halogen, Cyano, jeweils geradkettiges oder verzweigtes Alkyl mit 1 bis 4 Kohlenstoffatomen sowie jeweils geradkettiges oder verzweigtes Halogenalkyl mit jeweils 1 bis 4 Kohlenstoffatomen und 1 bis 9 gleichen oder verschiedenen Halogenatomen;
außerdem für jeweils gegebenenfalls einfach oder mehrfach, gleich oder verschieden substituiertes Aryl mit 6 bis 10 Kohlenstoffatomen oder Heteroaryl mit 2 bis 9 Kohlenstoffatomen und 1 bis 4 gleichen oder verschiedenen Heteroatomen - insbesondere Stickstoff, Sauerstoff und/oder Schwefel - steht, wobei als Substituenten jeweils infrage kommen:
Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen; zweifach verknüpftes, geradkettiges oder verzweigtes, gegebenenfalls ein- bis vierfach, gleich oder verschieden durch Halogen substituiertes Dioxyalkylen mit 1 bis 3 Kohlenstoffatomen; Dialkylamino, N-Alkanoylamino, Alkoxycarbonyl oder Alkoximinoalkyl mit jeweils 1 bis 4 Kohlenstoffatomen in den einzelnen geradkettigen oder verzweigten Alkylteilen sowie jeweils gegebenenfalls einfach oder mehrfach, gleich oder verschieden durch Halogen und/oder geradkettiges oder verzweigtes Alkyl mit 1 bis 4 Kohlenstoffatomen substituiertes Aryl oder Aryloxy mit 6 oder 10 Kohlenstoffatomen und

n für eine Zahl 0, 1, 2 oder 3 steht;

wobei jedoch Verbindungen, bei denen gleichzeitig $R^1$ für einen Methylrest, X für Sauerstoff, Y für Sauerstoff und $R^2$ für einen Rest der Formel

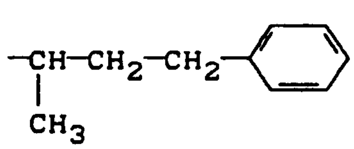 ; 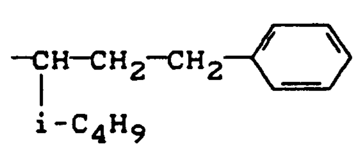 ;

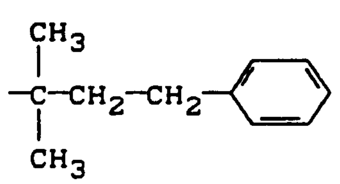 oder 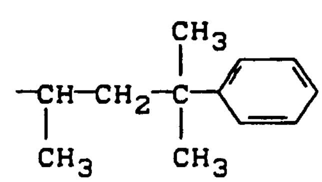

steht, ausgenommen sind.

**3.** Substituierte Triazolinone der allgemeinen Formel (I) gemäß Anspruch 1,

bei welchen

$R^1$ für Methyl, Ethyl, n- oder i-Propyl, n-, i-, s-oder t-Butyl, Cyclopropyl, Cyclobutyl, Cyclopentyl oder Cyclohexyl steht,

$R^2$ für jeweils gegebenenfalls ein-bis zweifach, gleich oder verschieden substituiertes Cyclopropyl, Cyclobutyl, Cyclopentyl oder Cyclohexyl steht, wobei als Substituenten jeweils infrage kommen:
im Arylteil jeweils gegebenenfalls ein-bis dreifach, gleich oder verschieden substituiertes Phenyl, Benzyl, Phenylethyl, Phenylpropyl, Phenylethenyl, Phenylpropenyl, Phenylethinyl, Phenylpropinyl, α-Naphthyl oder β-Naphthyl, wobei als Arylsubstituenten jeweils infrage kommen:
Fluor, Chlor, Brom, Cyano, Nitro, Methyl, Ethyl, n- oder i-Propyl , n-, i-, s-oder t-Butyl, Methoxy, Ethoxy, n- oder i-Propoxy, n-, i-, s-oder t-Butoxy, Methylthio, Ethylthio, Trifluormethyl, Trifluormethoxy, Trifluormethylthio, Methoxycarbonyl, Ethoxycarbonyl, Methoximinomethyl, Methoximinoethyl, Ethoximinomethyl, Ethoximinoethyl oder

gegebenenfalls ein- bis dreifach, gleich oder verschieden durch Fluor, Chlor, Brom, Methyl und/oder Ethyl substituiertes Phenyl,

$R^2$ außerdem für einen Rest der Formel

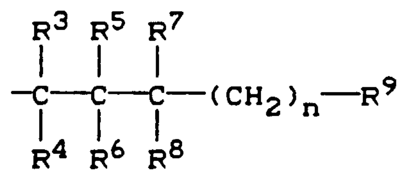

steht,

X für Sauerstoff oder Schwefel steht und

Y für Sauerstoff oder Schwefel steht, wobei

$R^3$ für geradkettiges oder verzweigtes Alkyl mit 1 bis 6 Kohlenstoffatomen steht,

$R^4$, $R^5$, $R^6$, $R^7$ und $R^8$ für Wasserstoff oder für geradkettiges oder verzweigtes Alkyl mit 1 bis 3 Kohlenstoffatomen stehen,

$R^9$ für jeweils gegebenenfalls ein-bis fünffach, gleich oder verschieden substituiertes Cyclopropyl, Cyclobutyl, Cyclopentyl oder Cyclohexyl steht, wobei als Substituenten jeweils infrage kommen:
Fluor, Chlor, Brom, Cyano, Methyl, Ethyl, n-oder i-Propyl, Chlormethyl, Dichlormethyl, Trichlormethyl, Difluormethyl oder Trifluormethyl;
außerdem für jeweils gegebenenfalls ein-bis dreifach, gleich oder verschieden substituiertes Phenyl, α-Naphthyl, β-Naphthyl, Pyridyl, Pyrimidyl, Triazinyl, Pyridazinyl, Pyrazolyl, Imidazolyl, Triazolyl, Thiazolyl, Isothiazolyl, Benzthiazolyl, Oxazolyl, Isoxazolyl, Benzoxazolyl, Benzopyrazolyl, Pyrrolyl, Furanyl, Thienyl, Indoyl, Benzopyranyl oder Chinolyl steht, wobei als Substituenten jeweils infrage kommen:
Fluor, Chlor, Brom, Cyano, Nitro, Methyl, Ethyl, n- oder i-Propyl, n-, i-, s-oder t-Butyl, Methoxy, Ethoxy, n- oder i-Propoxy, n-, i-, s- oder t-Butoxy, Methylthio, Ethylthio, Difluormethyl, Trifluormethyl, Difluormethoxy, Trifluormethoxy, Difluormethylthio, Trifluormethylthio, Dimethylamino, Diethylamino, N-Acetamido, Dioxymethylen, Difluordioxymethylen, Dioxyethylen, Trifluordioxyethylen, Tetrafluordioxyethylen, Methoxycarbonyl, Ethoxycarbonyl, Propoxycarbonyl, Methoximinomethyl, Methoximinoethyl, Ethoximinomethyl, Ethoximinoethyl oder gegebenenfalls ein- bis dreifach, gleich oder verschieden durch Fluor, Chlor, Brom, Methyl, Ethyl, Methoxy und/oder Ethoxy substituiertes Phenyl, Phenoxy, α-Naphthyl oder β-Naphthyl und

n für eine Zahl 0, 1 oder 2 steht,

wobei jedoch Verbindungen, bei denen gleichzeitig $R^1$ für einen Methylrest, X für Sauerstoff, Y für Sauerstoff und $R^2$ für einen Rest der Formel

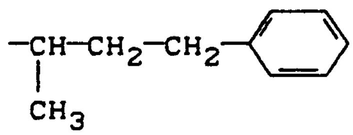 ; 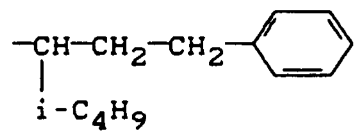 ;

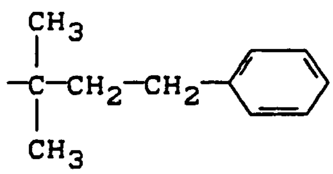 oder 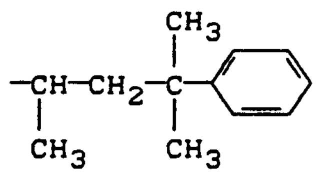

steht, ausgenommen sind.

**4.** Substituierte Triazolinone der allgemeinen Formel (I) gemäß Anspruch 1, bei welchen

$R^1$ für Methyl, Ethyl, n- oder i-Propyl oder für Cyclopropyl steht,

$R^2$ für einen Rest der Formel

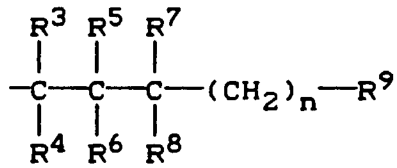

steht,

X für Sauerstoff steht,

Y für Sauerstoff steht,

$R^3$ für Methyl, Ethyl, n- oder i-Propyl, n-, i-, s-oder t-Butyl steht,

$R^4$, $R^5$, $R^6$, $R^7$ und $R^8$ unabhängig voneinander jeweils für Wasserstoff, Methyl oder Ethyl stehen,

$R^9$ für gegebenenfalls ein- bis dreifach, gleich oder verschieden substituiertes Phenyl steht, wobei als Substituenten jeweils infrage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, Difluormethyl, Trifluormethyl, Difluormethoxy, Trifluormethoxy, Difluormethylthio, Trifluormethylthio, Dimethylamino, Diethylamino, N-Acetamido, Dioxymethylen, Difluordioxymethylen, Dioxyethylen, Trifluordioxyethylen, Tetrafluordioxyethylen, Methoxycarbonyl, Ethoxycarbonyl, Propoxycarbonyl, Methoximinomethyl, Methoximinoethyl, Ethoximinomethyl, Ethoximinoethyl oder jeweils gegebenenfalls ein-bis dreifach, gleich oder verschieden durch Fluor, Chlor, Brom, Methyl, Ethyl, Methoxy und/oder Ethoxy substituiertes Phenyl, Phenoxy, α-Naphthyl oder β-Naphthyl und

n für eine Zahl 0 oder 1 steht,

wobei jedoch Verbindungen, bei denen gleichzeitig $R^1$ für einen Methylrest, X für Sauerstoff, Y für Sauerstoff und $R^2$ für einen Rest der Formel

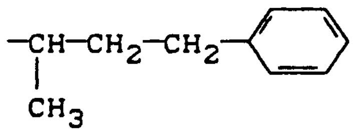 ; 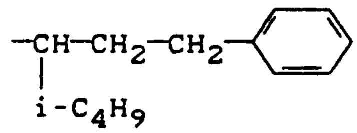 ;

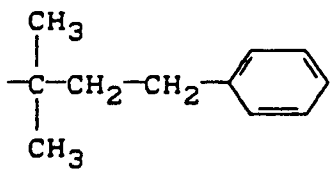 oder 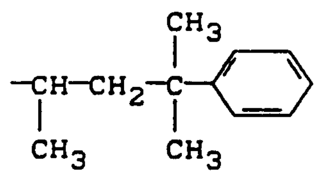

steht, ausgenommen sind.

**5.** Verfahren zur Herstellung von substituierten Triazolinonen der allgemeinen Formel (I) gemäß Anspruch 1,

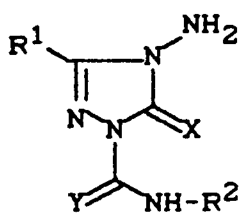

(I)

in welcher

| | |
|---|---|
| $R^1$ | für Alkyl oder Cycloalkyl steht, |
| $R^2$ | für gegebenenfalls durch Aryl, Arylalkyl, Arylalkenyl oder Arylalkinyl substituiertes Cycloalkyl steht, oder für einen Rest der Formel |

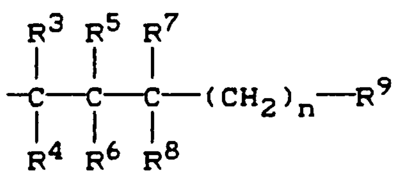

steht,

| | |
|---|---|
| X | für Sauerstoff oder Schwefel steht und |
| Y | für Sauerstoff oder Schwefel steht, |

wobei

| | |
|---|---|
| $R^3$, $R^4$, $R^5$, $R^6$, $R^7$ und $R^8$ | für Wasserstoff oder Alkyl stehen, |
| $R^9$ | für jeweils gegebenenfalls substituiertes Cycloalkyl, Aryl oder Heteroaryl steht und |
| n | für eine Zahl 0, 1, 2 oder 3 steht, |

wobei jedoch Verbindungen, bei denen gleichzeitig $R^1$ für einen Methylrest, X für Sauerstoff, Y für Sauerstoff und $R^2$ für einen Rest der Formel

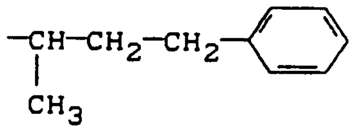 ; 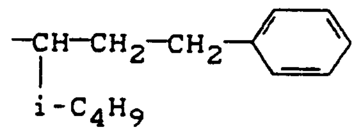 ;

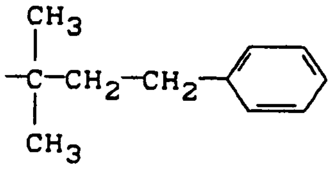 oder 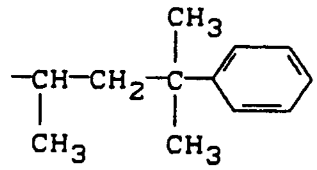

steht, ausgenommen sind.

dadurch gekennzeichnet, daß man

a) Hydrazone der Formel (II),

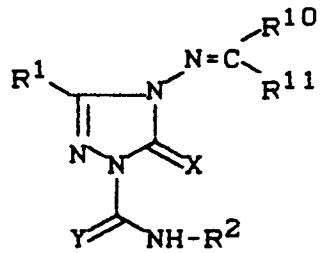

(II)

in welcher

$R^1$, $R^2$, X und Y die oben angegebene Bedeutung haben und

$R^{10}$ und $R^{11}$ unabhängig voneinander jeweils für Wasserstoff, Alkyl, Aralkyl oder Aryl stehen,

mit einer Säure gegebenenfalls in Gegenwart eines Verdünnungsmittels umsetzt,

oder daß man

b) 1H-Triazolinone der Formel (III),

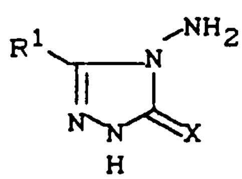

(III)

in welcher

$R^1$ und X die oben angegebene Bedeutung haben,

mit Iso(thio)cyanaten der Formel (IV),

$R^2$-N = C = Y (IV)

in welcher

$R^2$ und Y die oben angegebene Bedeutung haben,

gegebenenfalls in Gegenwart eines Verdünnungsmittels und gegebenenfalls in Gegenwart eines Reaktionshilfsmittels umsetzt,

oder daß man

c) Triazolinone der Formel (V),

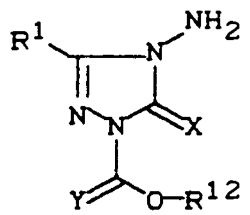

(V)

in welcher

$R^1$, X und Y die oben angegebene Bedeutung haben und

$R^{12}$ für Alkyl, Aralkyl oder Aryl steht,

mit Aminen der Formel (VI),

$R^2$-$NH_2$ (VI)

in welcher

$R^2$ die oben angegebene Bedeutung hat,

gegebenenfalls in Gegenwart eines Verdünnungsmittels und gegebenenfalls in Gegenwart eines Reaktionshilfsmittels umsetzt.

**6.** Herbizide Mittel, gekennzeichnet durch einen Gehalt an mindestens einem substituierten Triazolinon der Formel (I) gemäß den Ansprüchen 1 bis 5.

**7.** Verfahren zur Bekämpfung von unerwünschten Pflanzen, dadurch gekennzeichnet, daß man substituierte Triazolinone der Formel (I) gemäß den Ansprüchen 1 bis 5 auf unerwünschte Pflanzen und/oder ihren Lebensraum einwirken läßt.

**8.** Verwendung von substituierten Triazolinonen der Formel (I) gemäß den Ansprüchen 1 bis 5 zur Bekämpfung von unerwünschten Pflanzen.

**9.** Verfahren zur Herstellung von herbiziden Mitteln, dadurch gekennzeichnet, daß man substituierte Triazolinone der Formel (I) gemäß den Ansprüchen 1 bis 5 mit Streckmitteln und/oder oberflächenaktiven Substanzen vermischt.

**10.** Substituierte Hydrazone der Formel (II)

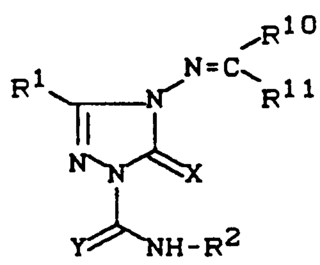

(II)

in welcher

$R^1$ für Alkyl oder Cycloalkyl steht,

$R^2$ für gegebenenfalls durch Aryl, Arylalkyl, Arylalkenyl oder Arylalkinyl substituiertes Cycloalkyl steht, oder für einen Rest der Formel

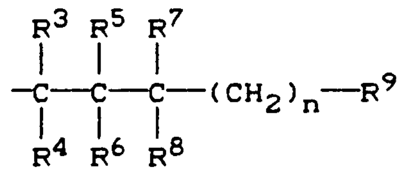

steht,

X für Sauerstoff oder Schwefel steht und

Y für Sauerstoff oder Schwefel steht,

wobei

$R^3$, $R^4$, $R^5$, $R^6$, $R^7$ und $R^8$ für Wasserstoff oder Alkyl stehen,

$R^9$ für jeweils gegebenenfalls substituiertes Cycloalkyl, Aryl oder Heteroaryl steht und

n für eine Zahl 0, 1, 2 oder 3 steht und

$R^{10}$ und $R^{11}$ unabhängig voneinander jeweils für Wasserstoff, Alkyl, Aralkyl oder Aryl stehen.

European Patent Office

# EUROPEAN SEARCH REPORT

Application Number

EP 92 10 6779

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
| --- | --- | --- | --- |
| A | EP-A-0 370 293 (BAYER) *Insgesamt* | 1-10 | C07D249/12<br>A01N43/653 |
| A | EP-A-0 399 294 (BAYER) *Insgesamt* | 1-10 | |
| D,A | EP-A-0 294 666 (BAYER) *Insgesamt* | 1-10 | |

TECHNICAL FIELDS SEARCHED (Int. Cl.5)

C07D

The present search report has been drawn up for all claims

| Place of search | Date of completion of the search | Examiner |
| --- | --- | --- |
| THE HAGUE | 21 JULY 1992 | LUYTEN H.W. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)